# EUROPEAN PATENT SPECIFICATION

(11) **EP 3 371 598 B1**
(45) Date of publication and mention of the grant of the patent: **14.07.2021**
(21) Application number: 16794257.2
(22) Date of filing: 04.11.2016
(51) Int. Cl.: G01N 33/569, G16B 20/00

(54) **DETERMINING PREDICTED INDIRECTLY RECOGNIZED HLA-DERIVED PEPTIDES**
BESTIMMUNG VON VORHERGESAGTEN INDIREKT ANERKANNTEN PEPTIDEN AUS HLA
DÉTERMINATION DE PEPTIDES DÉRIVÉS DE HLA RECONNUS INDIRECTEMENT PRÉDITS

(30) Priority: 05.11.2015 EP 15193290
(43) Date of publication of application: 12.09.2018
(73) Proprietor: PIRCHE AG, 10785 Berlin (DE); UMC Utrecht Holding B.V., 3584 CM Utrecht (NL)
(72) Inventor: SCHLIEHE-DIECKS, Ralf, 15366 Hoppegarten (DE); NIEMANN, Matthias, 10627 Berlin (DE); SPIERINGS, Eric, 3705 EG Zeist (NL)
(74) Representative: Hertin und Partner Rechts- und Patentanwälte PartG mbB
(86) International application number: PCT/EP2016/076653
(87) International publication number: WO 2017/077026

(56) References cited:
- WO-A1-2015/169597
- US-A1- 2015 278 434
- K. GENEUGELIJK ET AL: "Predicted Indirectly Recognizable HLA Epitopes Presented by HLA-DRB1 Are Related to HLA Antibody Formation During Pregnancy", AMERICAN JOURNAL OF TRANSPLANTATION, vol. 15, no. 12, 29 October 2015 (2015-10-29), pages 3112-3122, XP055333276, DK ISSN: 1600-6135, DOI: 10.1111/ajt.13508

### SEQUENCE LISTING

<110> PIRCHE AG
<120> METHOD AND DATA STRUCTURE FOR DETERMINING PREDICTED INDIRECTLY RECOGNIZED HLA-DERIVED PEPTIDES
<130> XII 2319/15WO
<150> EP15193290.2
   <151> 2015-11-05
<160> 10
<170> PatentIn version 3.5
<210> 1
   <211> 9
   <212> PRT
   <213> Homo sapiens
<400> 1
   Met Ala Val Met Ala Pro Arg Thr Leu
   1               5
<210> 2
   <211> 9
   <212> PRT
   <213> Homo sapiens
<400> 2
   Met Met Val Leu Gln Val Ser Ala Ala
   1               5
<210> 3
   <211> 15
   <212> PRT
   <213> Homo sapiens
<400> 3
   Arg Ala Tyr Leu Glu Gly Thr Cys Val Asp Gly Leu Arg Arg Tyr
   1               5                   10                  15
<210> 4
   <211> 15
   <212> PRT
   <213> Homo sapiens
<400> 4
   His Asn Tyr Gln Leu Glu Leu Arg Thr Thr Leu Gln Arg Arg Val
   1               5                   10                  15
<210> 5
   <211> 15
   <212> PRT
   <213> Homo sapiens
<400> 5
   Asn Tyr Gln Leu Glu Leu Arg Thr Thr Leu Gln Arg Arg Val Glu
   1               5                   10                  15
<210> 6
   <211> 9
   <212> PRT
   <213> Homo sapiens
<400> 6
   Tyr Gln Leu Glu Leu Arg Thr Thr Leu
   1               5
<210> 7
   <211> 9
   <212> PRT
   <213> Homo sapiens
<400> 7
   Arg Met Arg Arg His Asn Tyr Glu Leu
   1               5
<210> 8
   <211> 9
   <212> PRT
   <213> Homo sapiens
<400> 8
   Met Arg Val Met Ala Pro Arg Ala Leu
   1               5
<210> 9
   <211> 9
   <212> PRT
   <213> Homo sapiens
<400> 9
   Arg Val Met Ala Pro Arg Ala Leu Leu
   1 5
<210> 10
   <211> 9
   <212> PRT
   <213> Homo sapiens
<400> 10
   Val Met Ala Pro Arg Ala Leu Leu Leu
   1 5

## Description

The invention relates to a method and system for carrying out a computer implemented method for determining the numbers of predicted indirectly recognized HLA epitopes (PIRCHES) between one or more donors and one or more recipients of transplantation material. The method and system described herein allow the identification of permissible mismatches and therefore the provision of relatively safe transplantation material via in silico analysis of HLA-derived peptides of patient and donor.

This invention therefore relates to practical embodiments of a computer-implemented method that electronically simulates biological phenomena in a novel data structure or data source in a fast and reliable method. The novel data structure enables the development and utilisation of interactive software modules that determine of the number of recipient-specific (or donor-specific) HLA-derived peptides from a mismatched recipient-HLA (or donor-HLA) allele that are predicted to be presented by a shared/matched (or any MHC class II) HLA molecule (PIRCHES).

In particular, the present invention relates to the use of the data structure and methods based thereon as described herein for predicting or assessing the risk of an unwanted host versus graft immune reaction, for example in the context of solid organ transplant, on the basis of determining the number of PIRCHE-II. In this embodiment the invention encompasses the determination of the number of donor-specific HLA-derived peptides from a mismatched donor-HLA allele that are predicted to be presented by any MHC class II HLA molecule (PIRCHE-II).

Large numbers of PIRCHES are associated with an increased risk of an unwanted immune reaction after transplantation of donor material. The prediction of the numbers of PIRCHES in a fast and efficient manner therefore enables an improved selection of safe transplantation material. Furthermore, the invention specifies how the functionality shall be integrated in existing HLA matching (for example CordMatch) applications.

### BACKGROUND OF THE INVENTION

Transplantation of allogeneic cells, tissues and organs is an evolving therapy that has become an increasingly attractive therapeutic option. The number of patients receiving transplants from unrelated donors is expected to double in the near future. Alloreactivity after transplantation has a major impact on clinical outcome, with pathological as well as beneficial effects. HLA mismatches are known to induce an immune reaction after transplantation, however the factors involved in predicting risk of unwanted immune reaction are not well understood.

Hematopoietic Stem Cell Transplantation (HSCT) is one example of a quickly growing therapeutic approach. The major limiting factor of HSCT remains the risk of graft-versus-host disease (GVHD), and since the number of patients receiving HSCT is expected to increase, the provision of novel approaches to prevent GVHD must be accelerated. To overcome the risk of GVHD, patients are preferably transplanted with a donor that is completely matched for all HLA-alleles. However, due to diversity of HLA molecules in the population, these completely matched donors are not available for approximately 40% of patients. When a completely matched donor is not available, a clinician often has to face the difficult decision to choose the best donor out of the mismatched donors (i.e. the one that carries the lowest GVHD risk).

Until now, determining which donor is most suitable relies on a laborious assay that requires up to 14 days of lab-work, for example the cytotoxic T-lymphocyte precursor frequency (CTLpf) assay. Functionally, better-permissible mismatches can be determined with the CTLpf assay. CTLpf scores of less than or equal to 1 per 10⁶ PBL is associated with a better overall survival (Heemskerk et al (2007) Bone Marrow Transplantation, 40, 193-200). Despite such useful information able to be provided by laboratory methods, the time required for the analysis is prolonged and may lead to further detriment or death in patients in need of transplantation.

Similar problems face clinicians before selecting transplantation material for cord blood or cord blood cell transplantations, kidney transplantations, or other transplantations at risk of side effects caused by unwanted alloreactivity. To find an alternative for the CTLpf assay, multiple, so far unsuccessful, attempts have been undertaken to predict non-permissible mismatches using two generally available prediction programs, HLAMatchmaker and HistoCheck.

HLAMatchmaker determines potential epitopes for antibodies and has proven its validity for solidorgan transplantation (Duquesnoy et al, Hum. Immunol. 2002; 63: 353-63; Duquesnoy et al, Transplantation 2003; 75: 884-89). HLAMatchmaker considers differences in amino-acid triplets as epitopes on HLA. Although antibodies potentially play a role in the development of GVHD, predictions based on HLAMatchmaker are not correlated to alloreactivity (Gupta et al, Blood 2010; 1 16: 1839-48). As has been shown previously, the determination of PIRCHE represents a unique method for predicting unwanted immune responses during solid organ transplantation, such as kidney transplantation, when compared to HLA matchmaker (Otten et al., Hum Immunol. 2013 Mar;74(3):290-6.). US2015/0278434 discloses a method for prediction of an immune response against human leukocyte antigens (HLA) after transplantation, comprising a determination of the number of predicted indirectly recognized HLA epitopes (PIRCHES).

HistoCheck is based on the concept of direct recognition of HLA disparities, that is, donor T cells recognize an intact mis-matched-HLA molecule loaded with a non-polymorphic peptide (Amir et al, Blood 2011; 118: 6733). HistoCheck determines the structural differences in HLA molecules in the peptide-binding grooves or regions contacting the T-cell receptor (Elsner et al, Bone Marrow Transplant. 2004; 33:165- 69). By determining these structural differences, it aims to predict the likelihood of direct recognition of HLA disparities. Dissimilarity scores obtained with HistoCheck are also not correlated to alloreactivity (Spellman et al, Biol. Blood Marrow Transplant, 2011; Askar et al, Biol. Blood Marrow Transplant. 2011; 17: 1409-15). No clear evidence exists that HistoCheck is a reliable indicator for unwanted immune responses in solid organ transplantation.

In light of the previously existing techniques there exists a need for more reliable and faster methods for predicting whether donor material for a transplantation, which is HLA mismatched, is at increased risk of leading to a failed transplantation, for example development of GVHD, antibody mediated rejection (AMR), and/or an increase in mortality.

### SUMMARY OF THE INVENTION

In light of the prior art the technical problem underlying the present invention is the provision of improved or alternative computer-implemented means for the selection of donor cells or tissue preparations suitable for allogeneic transplantation with a low risk of adverse reaction, or for the prediction of an unwanted immune response in patients undergoing transplantation procedures, in particular via the determination of the number of PIRCHES between any given donor and recipient pair.

This problem is solved by the features of the independent claims. Preferred embodiments of the present invention are provided by the dependent claims.

The invention therefore relates to a computer implemented method for determining the numbers of predicted indirectly recognized HLA epitopes (PIRCHES) between one or more donors and one or more recipients of solid organ transplantation material,
wherein said PIRCHES are donor-specific HLA-derived peptides from a mismatched donor-HLA allele and are predicted to be presented by any MHC class II HLA molecule, wherein said method comprises:
a) Identification of peptides from mismatched HLA molecules of the donor;
b) Determination of the peptides identified in a) that are predicted to be presented by any recipient's MHC class II HLA molecule;
c) Identification of peptides from all selected HLA molecules of the recipient;
d) Determination of the peptides identified in c) that are predicted to be presented by any recipient's MHC class II HLA molecule;
   **wherein**
e) any peptide determined in b) that is not included in the set of peptides in d) is characterized as a PIRCHE II;
   **characterised in that**
f) steps a) to d) are performed on information stored in a graph data structure,
   **wherein**
g) the graph data structure comprises:
   - one or more HLA value entities,
   - one or more peptide entities, and
   - one or more relationships between said HLA value and peptide entities, wherein each entity is present in a single instance within the graph data structure and may exhibit one or more relationships to another entity, and wherein the HLA value entities have relationships to peptides, but not to other HLA entities, and peptides have relationships to HLA values, but not to other peptides.

The method as described above may also be carried out with a different order of the method steps, for example:
1. Determine the recipient's HLA derived peptides that are likely to be cleaved. If no appropriate score prediction method for cleavage is available, then every possible peptide (preferably but not exclusively 15mer) will be determined,
2. Determine the donor's HLA derived peptides that do not match with the patient (derived from mismatched HLA alleles), by determining all peptides that are likely to be cleaved. If no appropriate score prediction method for cleavage is available, then every possible (preferably but not exclusively 15mer) peptide will be determined,
3. Determine all of the donor's peptides determined in (2) that were not found in (1), thereby providing the mismatched donor-specific HLA derived peptides,
4. Determine the binding of the peptides of (3) to any given MHC class II HLA molecule of the recipient, wherein PIRCHEs are all peptides of (3) that have a sufficient binding affinity (an IC50 score as can be set appropriately, for example by setting a parameterized threshold).

In one embodiment the analysis of the HvG direction, for example for solid organ transplantation material or other transplantation material for which a host versus graft immune response is the unwanted immune response that the recipient is at risk of, the method does not comprise the analysis of PIRCHE-I, rather only of PIRCHE-II. In a further embodiment of the HvG-analysis the analysis of predicted cleavage frequency, such as a prediction of proteasome cleavage likelihood, is not carried out. The analysed peptides are then preferably any given peptide derived from HA alleles of a length to be determined as desired, preferably 15mers.

Also disclosed is a graph data structure as described herein as such, in addition to its use in a method for determining the number of PIRCHES in any given corresponding method. The data structure may be used in any method step of the invention described herein, as is required or desired. The invention also relates to computer software for execution of the method and/or running of the system as described herein. In one embodiment the method as described herein is characterised in that step f) is carried out by software, for example one or more software modules.

A further embodiment of the invention relates to a system for selecting and/or screening donor material for transplantation, for example for selecting donor material with permissible mismatches from HLA mismatched unrelated donors, comprising software modules for HLA matching, and PIRCHE matching according to the method as described herein.

In one embodiment the method as described herein is characterised in that the information stored in the graph data structure is an electronically-stored representation of biological data, or corresponds to, or is an abstraction of, one or more biological entities. The present invention therefore relates to a computer implemented method in which data are processed that correspond to "real-world" biological entities. The graph data structure is not only characterized by the particular architecture of the data structure, but preferably also by the particular biological context of the entities and relationships embodied by the structure.

The novel graph data structure disclosed herein and its use in the method described herein enables significantly improved (shortened) processing times in comparison to those approaches attempted previously. It was entirely surprising that the analysis of PIRCHE between donor and recipient could be enhanced to such a significant extent through the data structure employed in the present invention.

In one embodiment the method as described herein is characterised in that HLA matching between donor and recipient has been conducted in advance of carrying out the method described herein. The HLA-typing data of the method may have been carried out in advance and the data regarding HLA-type subsequently analysed via the method of the present invention.

In one embodiment the method as described herein is characterised in that the peptides from HLA molecules of the recipient and the peptides from HLA molecules of the donor are identified by a computer-implemented method for predicting the cleavage sites of the human proteasome within said HLA molecule(s). This refers preferably to steps a) and c) of the method described herein. The cleavage sites relate therefore preferably to endopeptidase and/or protease sites recognised by the human proteasome. A preferred embodiment of this feature of the invention relates to the use of the software NetChop, or alternative software as described herein, which is capable of determining proteasome cleavage of peptide sequences.

In one embodiment the method as described herein is characterised in that steps b) and d) of the method described above are performed using a computer-implemented method for predicting the binding of said peptide to any given HLA molecule. A preferred embodiment of this feature of the invention relates to the use of the software NetMHCpan and/or NetMHCII, or other alternatives as described herein, which are capable of determining (or predicting) the binding of any given, preferably nonameric, peptide in HLA or MHC molecules.

Other binding criteria may be applied for predicting whether a peptide will be presented by the HLA molecule. The provided values are based upon binding properties previously described in the literature, but different values may be derived or applied, if the PIRCHE can be reasonably be considered to bind the presenting HLA. The preferred values mentioned herein work surprisingly well. For each donor-recipient pair, presentable recipient- or donor-specific HLA-derived peptides (PIRCHES) are identified.

In one embodiment, per presenting HLA allele, predicted binders derived from donor-HLA alleles are regarded as donor-self peptides, and recipient-HLA alleles regarded as recipient-self peptides, depending on the therapeutic setting, and thus excluded from the analyses. In general, for each donor-recipient pair, the number of presentable recipient- or donor-specific peptides (derived from the mismatched recipient- or donor-HLA allele and predicted to be presented by shared or any MHC class II HLA molecules) is counted in order to generate the number of PIRCHES.

In particular, the PIRCHE value for HvG (antibody recognition) is the number of peptides obtained from the donor HLA molecules that do not match any peptide derived from the patient (recipient), and are likely to be presented by the patient's MHC class II HLA molecules.

PIRCHE-I may preferably be identified in two steps:
1) In silico assessment of predicted processing of the amino acid sequences by the proteasome and transportation via the TAP channel is carried out, preferably using NetChop. NetChop is a commonly known software-based method for identifying the cleavage sites of the human proteasome based on sequence analysis. Nonameric peptides are preferably included in the analyses. In a preferred embodiment, the C-terminal cleavage potential is determined. The entire HLA protein is cleaved (in silico) and all positions that are cleavable are marked. From the marked positions backwards, preferably nonameric peptides are identified that are analysed for binding to class I.
   As alternatives to NetChop a variety of software-based approaches are known in the art that could be applied, such as MAPP, PaProc or those methods described in Lu et al (J Zhejiang Univ Sci B, 2013 Sep; 14(9):816-28) or Ginodi et al (Bioinformatics, 2008 Feb 15;24(4):477-83).
2) Subsequently, peptides with a high probability of being processed (according to step 1) are tested for their capacity to be presented by HLA that are shared/matched between the donor and recipient (preferably HLA-A, -B and -C) using NetMHCpan. NetMHCpan is a commonly known method for identifying and/or predicting the binding of peptides to any known MHC molecule using artificial neural networks. The method is trained on more than 150,000 quantitative binding data covering more than 150 different MHC molecules. Predictions can be made for HLA-A, B, C, E and G alleles. The prediction values can be given in nM IC50 values, or as %-Rank to a set of 200000 random natural peptides. Preferably peptides with IC50 binding values <500nM are chosen as relevant binders.

PIRCHE-II may be identified as follows:
Preferably nonameric binding cores of potential 15-meric HLA-DR, -DQ, and -DP binders may preferably be analysed with NetMHCIIPan 2.0, NetMHCII 1 -0, or NetMHCII 2.2. Potential recipient- or donor-introduced peptides not represented by the donor- or patient-self-peptides having a high binding probability on shared/matched HLA class II molecules (or any of the recipient's HLA class II molecules) are considered as predicted indirectly recognizable HLA class 2 epitopes (PIRCHE-II). NetMHCII is a commonly known method for predicting binding of peptides to HLA-DR, HLA-DQ, HLA-DP and mouse MHC class II alleles using artificial neuron networks. Predictions can be obtained for 14 HLA-DR alleles covering the 9 HLA-DR supertypes, six HLA-DQ, six HLA-DP, and two mouse H2 class II alleles. The prediction values are given in nM IC50 values, and as a %-Rank to a set of 1000000 random natural peptides. Peptides with IC50-binding values <1000nM are considered as relevant.

There are a number of alternative methods known in the art, which could be used for determination of HLA binding. SYFPEITHI (based on methods described in Rammensee et al. Immunogenetics 41 , 178-228, 1995 and Rammensee et al, Landes Bioscience 1997) and BIMAS are well-known alternatives and are appropriate for class I binding but show some drawbacks in class II binding. Other alternatives relate to Tepitope (based on Stur-niolo et al, 1999, Nat. Biotechnol. 17:555-561), TepitopePAN, EpicCapo, PAAQD, POPI, Propred and Multipred.

In one embodiment the present invention relates to a method as described herein, wherein PIRCHE-I peptides have a predicted IC50 binding value of <10 µM, preferably <1000nM, more preferably <500nM.

In one embodiment the present invention relates to a method as described herein, wherein PIRCHE-II peptides have a predicted IC50 binding value of <20 µM, preferably <5 µM, more preferably <1000nM.

The identification of HLA-derived peptides and their potential binding properties as described above are subsequently provided and/or analysed in the context of a graph data structure in the context of the method of claim 1 and the system of 7.

According to the present invention, and in computer science, the term "graph data structure" is a data type or structure that comprises a collection of nodes (also called vertices), and the connections between them, called edges. A graph data structure may associate to each edge some edge value, such as a numerical attribute.

According to the present invention the nodes of the data structure are preferably represented as HLA value entities and peptide entities, wherein the edges are preferably represented as relationships between HLA value and peptide entities.

Typical operations associated with graph data structures include "adjacent(G, x, y)", which tests whether there is an edge from node x to node y, "neighbors(G, x)", which lists all nodes y such that there is an edge from x to y, or "get_node_value(G, x)", which returns the value associated with the node x. Further operations are available and are capable, for example, of selecting or providing particular nodes defined by certain relationships as defined herein, for example when selecting those peptides to be considered as PIRCHES for any given donor-recipient pair. A skilled person is therefore capable of using a graph data structure as described herein, via various operations carried out in software programmes, in order to determine the desired information regarding the number of PIRCHES for any given donor-recipient pair.

The method of the invention as described herein is characterised in that the graph data structure comprises:
- one or more HLA value entities,
- one or more peptide entities, and
- one or more relationships between said HLA value and peptide entities, wherein each entity is present in a single instance within the graph data structure and may exhibit one or more relationships to another entity.

In a preferred embodiment the one or more HLA value entities relate preferably to an informational and/or computational representation of an HLA allele. In a preferred embodiment the HLA value entities relate to alleles that are defined by a unique protein sequence, whereby HLA alleles that show only DNA sequence differences without changes in the corresponding protein sequence are preferably not considered in the method. An example of such HLA alleles that show only DNA sequence differences relate to allele-specific features described in fields 3 and 4 of the standard HLA nomenclature system relating to synonymous DNA sequence changes or sequence changes outside a coding region.

In a preferred embodiment the one or more peptide entities relate preferably to an informational and/or computational representation of a peptide sequence derived from an HLA value entity, whereby the sequence of any given HLA allele may be cleaved via a computational approach to produce a peptide sequence of any given length from within the HLA protein sequence, wherein peptides are preferably 5 to 30, preferably 7 to 11, more preferably 9, or preferably 12 to 18, more preferably 15, amino acids in length.

According to the invention, the HLA value entities have relationships to peptides, but not to other HLA entities, and peptides have relationships to HLA values, but not to other peptides. Through this set of relationships a graph data structure is formed that enables efficient (fast) interrogation.

In one embodiment the method as described herein is characterised in that any given HLA value entity "has" a number of peptides (from 1 to n), wherein each peptide of (derived from) any given HLA value entity has a relationship to said HLA value entity. The graph data structure is therefore defined by the relationships between each HLA value entity to its connected peptide entities, said relationships being preferably defined by the predicted proteasome cleavage of the amino acid sequence of the corresponding HLA molecule and the likelihood of proteasome cleavage.

In one embodiment the method as described herein is characterised in that the peptides (and number thereof) of each HLA value entity are determined by the predicted proteasome cleavage of the amino acid sequence of the corresponding HLA molecule.

In one embodiment the method as described herein is characterised in that the likelihood of proteasome cleavage is expressed by the cleavage score from 0 to 1 (preferably between 0 and 1), wherein said score represents a property of the relationship between any given HLA value entity and peptide entity.

In a preferred embodiment the method as described herein is characterised in that steps a) and c) of claim 1 are carried out so that said identification of peptides is determined according to the cleavage score. In one embodiment a cleavage score (relationship property or attribute) of preferably > 0.5 (threshold may be from 0 to 1, preferably between 0.1 to 0.9, more preferably between 0.3 to 0.7, most preferably 0.5) for any given peptide is sufficient for peptide identification for said HLA molecule.

In one embodiment the method as described herein is characterised in that any given HLA value entity "presents" a number of peptides (from 0 to n), wherein each peptide presented by any given HLA value entity has a relationship to said HLA value entity. In one embodiment the method, or the graph data structure as such, as described herein is characterised in that the presentation of a peptide by an HLA value entity is determined by the predicted binding and presentation of said peptide by said HLA molecule.

In a preferred embodiment the likelihood of peptide presentation (such as predicted binding of said peptide and presentation on cell surface) is expressed by the IC 50 score, which represents a property of a relationship between any given HLA value entity and peptide entity. In one embodiment for steps b) and d) of claim 1 said determination of peptides is carried out using an IC 50 score threshold.

In a preferred embodiment the invention relates to a computer implemented method for the prediction of an unwanted immune reaction (in this embodiment preferably for HSCT, or other GVH reactions) by determining the numbers of predicted indirectly recognized HLA epitopes (PIRCHES) between one or more donors and one or more recipients of transplantation material, wherein said PIRCHES are recipient-specific HLA-derived peptides from a mismatched recipient-HLA allele and are predicted to be presented by a shared (matched) HLA molecule, wherein the method comprises:
a) Identification of peptides from mismatched HLA molecules of the recipient;
b) Determination of the peptides identified in a) that are predicted to be presented by one or more of the selected shared (matched) HLA molecules;
c) Identification of peptides from all selected HLA molecules of the donor;
**d)** Determination of the peptides identified in c) that are predicted to be presented by one or more of the selected shared (matched) HLA molecules;
   **wherein**
e) for each shared MHC Class I HLA molecule, any peptide determined in b) that is not included in the set of peptides in d) is characterized as a PIRCHE I, or
   for each shared MHC Class II HLA molecule, any peptide determined in b) that is not included in the set of peptides in d) is characterized as a PIRCHE II;
   **characterised in that**
f) steps a) to d) are performed on information stored in a graph data structure, **wherein**
   - one or more nodes of the graph data structure are HLA value entities comprising an informational and/or computational representation of the HLA molecules,
   - one or more nodes of the graph data structure are peptide entities comprising an informational and/or computational representation of the peptides, and
   - edges between HLA value entities and peptide entities represent relationships between the HLA molecules and the peptides,
   **and wherein**
   - a first relationship represents the likelihood that a proteasome cleavage of the HLA molecule yields the peptide, and
   - a second relationship represents the likelihood that the peptide binds to the HLA molecule.

In a preferred embodiment the invention relates to a computer implemented method for the prediction of an unwanted immune reaction by determining the numbers of predicted indirectly recognized HLA epitopes (PIRCHES) between one or more donors and one or more recipients of transplantation material, wherein said PIRCHES are donor-specific HLA-derived peptides from a mismatched donor-HLA allele and are predicted to be presented by any HLA class II molecule, wherein the method comprises:
a) Identification of peptides from mismatched HLA molecules of the donor;
b) Determination of the peptides identified in a) that are predicted to be presented by one or more of the recipient HLA class II molecules;
c) Identification of peptides from all selected HLA molecules of the recipient;
d) Determination of the peptides identified in c) that are predicted to be presented by one or more of the recipient HLA class II molecules;
   **wherein**
e) for each recipient MHC Class II HLA molecule, any peptide determined in b) that is not included in the set of peptides in d) is characterized as a PIRCHE II;
   **characterised in that**
f) steps a) to d) are performed on information stored in a graph data structure, **wherein**
   - one or more nodes of the graph data structure are HLA value entities comprising an informational and/or computational representation of the HLA molecules,
   - one or more nodes of the graph data structure are peptide entities comprising an informational and/or computational representation of the peptides, and
   - edges between HLA value entities and peptide entities represent relationships between the HLA molecules and the peptides,
   **and wherein**
   - a first relationship represents the likelihood that a proteasome cleavage of the HLA molecule yields the peptide, and
   - a second relationship represents the likelihood that the peptide binds to the HLA molecule.

In a preferred embodiment the invention relates to a method as described herein, characterised in that the method is used for the selection and/or screening of donor material from multiple potential donors for transplantation, wherein
a peptide is identified from an HLA molecule in steps a) and c) if the likelihood that a proteasome cleavage of the HLA molecule yields the peptide as represented by the first relationship is larger than a threshold value,
a peptide is predicted to be presented by an HLA molecule in steps b) and d) if the likelihood that the peptide binds to the HLA molecule as represented by the second relationship is larger than a threshold value,
and the donor material is selected for which the lowest number of PIRCHES has been determined.

In a preferred embodiment the invention relates to a method as described herein, characterised in that the likelihood of proteasome cleavage is expressed by a cleavage score from 0 to 1 and the threshold for the identification of the peptide in steps a) and c) is 0.5
the likelihood for the binding of a peptide to an HLA molecule is expressed by the IC 50 score and the threshold in steps b) and d) for an MHC Class I HLA molecule is 500 and for an MHC Class II HLA molecule 1000.

In one embodiment the method as described herein is characterised in that said threshold is preferably an IC 50 score of ≤500 for PIRCHE I and/or ≤1000 for PIRCHE II, wherein such scores are sufficient for determination of predicted presentation of said peptide.

In a preferred embodiment the selected HLA molecules are the HLA loci A*, B*, C*, DRB1* and DQB1*, and optionally DPB1* and DRB3/4/5*.

A further aspect of the invention relates to a system for selecting and/or screening donor material for transplantation, for example for selecting donor material with permissible mismatches from mismatched unrelated donors, comprising software modules adapted for HLA matching, and for performing the method for determining the numbers of PIRCHES as described herein, wherein said system comprises a graph data structure as described herein.

In a preferred embodiment the system for selecting and/or screening donor material for transplantation is characterised in that said HLA software module carries out HLA matching between donor and recipient HLA typing data on the basis of high resolution allele coding (e.g. A*02:01), preferably for HLA loci A*, B*, C*, DRB1* and DQB1*, and optionally DPB1* and DRB3/4/5*.

It is further preferred that said PIRCHE matching incorporates the HLA matching information generated by said HLA matching software module, such that the PIRCHE matching module preferably accepts a patient, a matching profile and list of HLA matched donors and preferably cord blood units (CBUs) and returns the input donor list enriched with the PIRCHE information to the service client.

In a preferred embodiment the system is characterized in that patient information and search profiles are provided via a shared service integration manager software module, wherein said shared service integration manager module is connected to both HLA matching and PIRCHE matching modules, such that patient information and search profiles are initially processed by said HLA matching software module, which subsequently provides a patient, a matching profile and list of HLA matched donors and preferably cord blood units (CBUs) via said shared manager module for analysis of PIRCHE numbers to said PIRCHE matching module.

The various embodiments of the system provide combined approaches towards HLA matching and PIRCHE matching, thereby providing a unique service to transplantation providers searching for transplantation material with low risk of causing unwanted allogenic reactions. The technical effect of the system described herein is not only the improved speed of interrogation of donor databanks, but also the provision of safer transplantation material, thereby increasing health of the patient population having received transplantations of allogenic material.

In one aspect of the invention the method or system as described herein is characterised in that the information corresponding to donor HLA value entities is obtained from one or more donor profiles. The donor profile may be termed as a "donor type", which relates to an electronic (theoretical or according to a real donor) representation of any given potential combination of HLA alleles, and/or haplotypes, that may occur in an individual subject. A donor profile or type is preferably stored in an additional data structure, database, library and/or simulation comprising all possible donor types as described above.

In a preferred embodiment the donor profiles are preferably stored in an additional data structure, database, library and/or simulation of the peptide and/or HLA value entities of any given one or more theoretical or virtual donors. In one embodiment preferably multiple donor profiles or donor types are stored in a donor data structure, database, library and/or simulation comprising preferably essentially all possible donors for essentially all possible genotypes or possible combinations of HLA alleles, and/or haplotypes.

The phrase "essentially all" relates in this context to a significant number of, preferably all, or every known, protein variant of the allele group. The method therefore can effectively determine whether a particular donor or allele group indeed is associated with a relatively low PIRCHE value and therefore present itself as a preferred selection. Because those skilled in the art are able to make a pre-selection of possible protein variants within any given allele group, either on the basis of the reliability of the stored data or on population frequencies of certain alleles, the method is not limited to analysis of all known protein sequences of an allele group. In one embodiment the method will encompass the analysis of essentially all known (or relevant) protein sequences within the allele group, in order to provide more definitive results.

In a preferred embodiment of this aspect the method or system of the invention is characterised in that said method and/or system enables searching with recipient information (corresponding preferably to a biological recipient in need of a transplantation) against one or more donor profiles, wherein the result (outcome) of said system and/or method provides information on the one or more (preferably multiple) best-matched mismatched donor profiles, preferably a 9/10 mismatched donor with a lowest possible number of PIRCHES.

In one embodiment the method or system of the invention is characterised in that said method and/or system is functionally connected to electronic registers of donor material (for example registers that do not have information stored on PIRCHES), wherein the outcome of the method and/or system of the preceding claim enables a query to be sent from an end-user of said method and/or system to said register for any given one or more best-matched mismatched donor material corresponding to said outcome. The invention preferably links the end user, for example a medical practitioner or transplant provider, with the stem cell or other donor material registers that comprise information on said donor material.

In one embodiment the method or system of the invention is characterised in that information representing the frequency of any given combination of HLA alleles (known for example as a haplotype or genotype) in any one or more human populations (such as age, gender, ethnicity, etc....) is incorporated in said profile for each potential HLA allele combination of the donor profile.

In one embodiment the method or system of the invention is characterised in that electronically stored HLA typing data for donor material typed with "low-" or "medium-" resolution HLA typing is converted via a computer-implemented method into a "high-" resolution-similar or analogous HLA typing status on the basis of the known population frequencies of any given combination of HLA alleles determined by said with "low-" or "medium-" resolution HLA typing, thereby enabling interrogation of HLA typing data previously obtained via "low-" or "medium-" resolution techniques using the method and/or system as described herein.

A large number of donor samples stored for transplantation are at present only typed at either "low" or "intermediate" resolution. When no completely matched donor material is evident, some of the mismatched donor material may also be suitable for transplantation. However, clinicians searching for matched donor material face a difficult task in selecting "low" or "intermediate" resolution typed material for transplantation, or for further high resolution typing before transplantation. Determining which donor is most likely to provide acceptable allogeneic material, or which mismatched allele group is most likely to be associated with a low risk of an adverse immune reaction, remains a challenge for clinicians when faced with "low" or "intermediate" resolution typed donor information. The present invention therefore enables conversion of "low-" or "medium-" resolution HLA typing to "high" resolution data.

Considering the enormous health cost to patients having suffered from unwanted immune responses after transplantation, in addition to relatively high costs for carrying out high resolution typing, methods for the prediction of safely transplantable material and for the prediction of which mismatched or potentially mismatched donors are associated with a lower risk of an unwanted immune reaction, are of paramount importance to the medical community. The method as described herein enables reduction of risk upstream of surgery (or treatment) and upstream of any high resolution typing that may be required, thereby avoiding substantial health and financial cost to patients, medical practitioners and institutions, respectively.

In one embodiment the method or system of the invention is characterised in that said method and/or system comprises a) conversion of HLA typing data (of preferably donors) from "low-" or "medium-" resolution HLA typing to "high-" resolution data, b) HLA matching of said donor information with recipient HLA information and c) PIRCHE matching according to the method and/or system described herein.

A further aspect of the invention relates to a method or system characterised in that information representing the frequency of any given combination of HLA alleles (known for example as a haplotype or genotype) in any one or more human populations (such as age, gender, ethnicity, etc....) is incorporated in said profile for each potential HLA allele combination of the donor profile, wherein preferably electronically stored HLA typing data for donor material typed with "low-" or "medium-" resolution HLA typing is converted via a computer-implemented method into a "high-" resolution-similar or analogous HLA typing status on the basis of the known population frequencies of any given combination of HLA alleles determined by said with "low-" or "medium-" resolution HLA typing, thereby enabling interrogation of HLA typing data previously obtained via "low-" or "medium-" resolution techniques using the method and/or system as described herein.

The transplantation material may relate to any given biological matter intended for transplantation. Such biological matter may relate to cells, tissue, blood, in particular cord blood, hematopoietic stem cells, or other progenitor or stem cell populations, or organs for transplantation.

The computer implementation of the invention enables an efficient, fast and reliable method for identification of potentially permissible donor material for allogeneic transplantation. The data processed by the software can be handled in a completely or partially automatic manner, thereby enabling in a preferred embodiment an automated computer-implemented method. Data regarding the HLA typing of donor and recipient, in addition to the number of PIRCHES determined for any donor-recipient pair, can be stored electronically and eventually maintained in appropriate databases. The invention therefore also relates to computer software capable of carrying out the method as described herein. The invention further relates to a preferably automated computer-implemented method for prediction of an immune response against human leukocyte antigens (HLA) after transplantation, wherein the number of PIRCHES is correlated with risk of an unwanted immune reaction.

The system preferably comprises one or more databases with information on all published HLA alleles. The database may be updated as new HLA allele sequences are published. Computer software, which could also be based on any given computing language, can be used for generating and/or updating the databases, in addition to calling the respective programmes required.

The invention further comprises a system for preferably pre-transplantation prediction of an immune response against human leukocyte antigens (HLA), which may occur after transplantation. The system may comprise computing devices, data storage devices and/or appropriate software, for example individual software modules, which interact with each other to carry out the method as described herein.

In one embodiment the system may comprise databanks or databases of a cord blood bank, whereby each sample is tested for HLA-type, and the information stored electronically. The system may also comprise a connection between an additional computing device, for example a device of a clinician, transplant centre, or hospital, in which the HLA-type data for the recipient is stored. Through a connection between the multiple databases, for example over the internet, the method of the invention can be carried out using appropriate software. HLA-types of multiple potential donor samples and the patient may be compared and the number of PIRCHE for any given donor-recipient pair determined. In light of the analysis based on the method described herein a clinically relevant prediction can be made whether any given donor material, for example those samples stored in a cord blood bank or other cell or tissue bank, is suitable for transplantation.

In one embodiment the present invention relates to a method as described herein, wherein HLA typing is carried out on HLA subtypes HLA-A, HLA-B, HLA-C, HLA-DRB1 , HLA-DQB1 , HLA-DPB1 , -DQA1 , -DPA1 , -G, -E, -F, MICA, MICB and/or KIR.

In one embodiment the present invention relates to a method as described herein, wherein HLA typing is carried out on HLA-A, -B, -C, -DRB1 and -DQB1. In the domain of stem cell transplantation, these 5 alleles provide the basis for the commonly used terminology "9/10-matched", which refers to a 9/10-matched unrelated donor. In such a case 9 of the 10 alleles of these 5 genes show a match but one remaining allele does not match. The present invention therefore allows, in a preferred embodiment, determination of whether the use of material from such a 9/10-matched unrelated donor is safe, i. e. whether the mismatch is permissible for transplantation. In the domain of solid organ transplantation, sometimes only a subset of the named HLA subtypes (HLA-A, HLA-B and HLA-DRB1) is focused on.

The invention relates also to the method as described herein for finding permissible HLA-mismatches in donor samples where more mismatches are present than the common 9/10 scenario. One example of potential donors who are encompassed by the present invention are Haploidentical donors, who may be screened using the method described herein for permissible mismatched donor material. A haploidentical related donor, may be described as a donor who has a "50% match" to the patient. This type of donor can be a parent, sibling, or child. By definition, a parent or child of a patient will always be a haploidentical donor since half of the genetic material comes from each parent. There is a 50% chance that a sibling will be a haploidentical donor. Haploidentical HSCT offers many more people the option of HCT as 90% of patients have a haploidentical family member. Other advantages include: immediate donor availability; equivalent access for all patients regardless of ethnic background; ability to select between multiple donors; and ability to obtain additional cells if needed. Alternatively, cord blood units (CBUs) may not show a high level of HLA-matching, but still be suitable for transplantation if the mismatches are permissible as determined by the method as described herein. CBU's are typically minimally 4/6 matched, but this match can however lead to a 4/10 or 5/10 situation at the allelic level.

Considering that in case of HSCT the method as described herein relies to some extent on shared HLA, the minimum HLA-match is one allelic match. The invention may therefore be carried out on mismatched samples with a minimum of one allelic match. The mismatched donor may therefore relate to a 1/10, 2/10, 3/10, 4/10, 5/10, 6/10, 7/10, 8/10, 9/10 or 10/10 (DP mismatch) match. If additional alleles are tested, the donor may also show any other kind of mismatch, whereby at least one allelic match is present. Preferred for the transplantation are donors with significant HLA-matches. If additional alleles are subjected to typing the donor could therefore be for example 11/12- or 13/14-matched. Restricting a patient-donor-pair to have at least a single allelic match is not required for the domain of solid organ transplantation or antibody recognition. The method as described herein relies on all of the patient's HLA class II molecules and is therefore independent of shared HLA.

In one embodiment the present invention relates to a method as described herein, wherein HLA typing comprises serological and/or molecular typing. In a preferred embodiment the present invention relates to a method as described herein, wherein HLA typing is carried out at high resolution level with sequence-based typing.

In one embodiment the present invention relates to a method as described herein, wherein HLA typing comprises sequencing of exon 1-7 for HLA class I alleles and exon 1-6 for HLA class II alleles. In one embodiment the present invention relates to a method as described herein, wherein HLA typing comprises high resolution HLA-A, -B, -C, -DRB1 and -DQB1 typing of exons 2 and 3 for HLA class-I alleles and exon 2 for HLA class-II alleles. These particular HLA-typing approaches are described in the examples provided herein and demonstrate advantages over earlier typing methods, providing full coverage of the alleles that need to be typed to identify the mismatches.

### DETAILED DESCRIPTION OF THE INVENTION

Historically, alloreactivity after HSCT is considered to be evoked mostly due to direct recognition of HLA disparities by donor T cells. This means that the graft T cells recognize mismatched HLA that is expressed as an intact molecule on the cell surface of host cells. The present invention is however based in particular upon indirect recognition. Alloreactivity can be evoked when peptides derived from the mismatched host HLA allele are processed and presented on shared HLA and thereby recognized by the donor T cells.

Analogous to HSCT, alloreactivity after solid organ transplantation is considered to be evoked mostly due to direct recognition of HLA disparities by patient T cells. The present invention is however based in particular upon indirect recognition of peptides introduced by the foreign tissue. Alloreactivity can be evoked by the mechanism of linked recognition. Donor cells from the transplanted material are recognized by the patient's B cells, which typically bind pathogens, using their B cell receptors. The B cells process the foreign MHC molecules (from transplanted organ material) and present peptides derived from these foreign MHC molecules on their MHC class II. The MHC class II-presented peptides, derived from foreign MHC molecules, can then be detected by the patient's (recipient's) T cells. The unwanted immune reaction in this case is based on a "Host vs Graft" immune response.

As the MHC molecules that are processed and presented are generated and subsequently detected by the patient's (recipient's) immune system, there is no requirement of presentation by shared HLA - all HLA class II molecules can be considered as presenting. In other words, any given HLA class II molecule of the recipient may present the cleaved MHC peptides of the transplantation (donor) material.

Binding of peptides to HLA molecules is predictable. The differences between predicted binding affinities and experimental measurements have been shown to be as small as the differences in measurements between different laboratories. Predictability is particularly high for HLA class-I molecules, as these molecules have a more strict preference for nine amino acid long peptides (9-mers) and require specific amino acids as anchor residues at clearly defined anchor positions. For HLA class II molecules predictability is lower, as peptides of different length can bind using different positions as anchor residues. Therefore, it is difficult to determine how a peptide aligns to the HLA class II-binding groove and which amino-acid residues in the peptide are preferred as anchors. To solve this problem, Nielsen et al. used a so-called core predictor to estimate how a peptide positions in the class II binding groove. The core predictor enabled the development of an accurate HLA class-II predictor, called NetMHCII.

Despite the advances in predicting peptide binding to class I and class II HLA molecules, there is still significant uncertainty in assessing the factors involved in alloreactivity and the production of donor-specific antibodies (DSA). Considering the tools presently available to predict unwanted immune responses after kidney transplantation, there exists a need to provide more reliable methods for assessing potentially adverse reactions in advance of transplantation.

Conceptually, mismatched HLA-directed T-cell alloreactivity may result from direct and indirect recognition of HLA disparities. So far, studies that aimed at explaining and predicting the clinical alloreactivity towards mismatched HLA, mainly focused on direct recognition of HLA disparities. Direct recognition involves donor T cells that recognize an intact mismatched-HLA molecule loaded with a non-polymorphic peptide. When polymorphisms in HLA alleles lead to differences in the peptide-binding groove, the presented peptide repertoire of HLA molecules may differ substantially. These different peptide repertoires may lead to T-cell responses. The HistoCheck algorithm determines structural differences in HLA molecules in the peptide-binding grooves or regions contacting the T-cell receptor, thereby predicting a dissimilarity score. However, the scores obtained with HistoCheck do not correlate with alloreactivity, neither in vitro, nor in vivo.

T-cell related alloreactivity can potentially also be evoked by indirect recognition of the mismatched-HLA allele. Indirect recognition has been studied in great detail for minor histocompatibility (H) antigens. Mismatches for these HLA-presented polymorphic proteins are associated with an increased risk of aGVHD, and a decreased risk of relapse. Analogous to peptides derived from minor H mismatches, peptides derived from mismatched-HLA molecules can also be presented by HLA.

Indirect recognition of the mismatched-HLA antigen may lead to T cell-related alloreactivity. During indirect HLA recognition, T cells recognize peptides derived from polymorphic HLA antigens presented by a shared/matched or any MHC class II HLA molecule. Peptides derived from mismatched-HLA molecules are frequently presented by HLA. These indirectly recognizable HLA epitopes have been associated with both acute and chronic graft failure in solid organ transplantation. T cells that indirectly recognize HLA-mismatches in the context of self-HLA may therefore play an important role in clinical alloreactivity.

The present invention therefore designates the HLA-derived epitopes that are predicted to be presented as Predicted Indirectly ReCognizable HLA Epitopes (PIRCHES). The present invention identifies PIRCHES presented by shared- HLA class-I (PIRCHE-I) and class-II (PIRCHE-II) separately. PIRCHE-II are shown to induce alloreactivity after kidney transplantation; PIRCHES presented by HLA-DR correlated with the de novo development of donor-specific HLA IgG antibodies.

The present invention is therefore based on the finding that recognition of HLA-derived peptides has an effect on clinical alloreactivity after HLA-mismatched HSCT. To this end, numbers of predicted PIRCHE-I and -II can be assessed and their role evaluated in the adverse clinical effects of HSCT. On the basis of such investigation, the present invention describes universally applicable methods that can predict non-permissible HLA mismatches prior to HSCT and other cell or organ transplants.

The approaches of the prior art, such as HLAMatchmaker, which assesses the degree of structural compatibility between mismatches, have not provided effective means for predictive determination of the risk of an unwanted immune reaction in the context of HSCT. HLAMatchmaker considers the structural basis of epitopes on HLA-antigens that could induce HLA-antibodies. It does so by looking at HLA class I antigens as a combination of short sequences (triplets, later eplets), and determining the differences in these triplets. For HSCT, the degree of triplet mismatching did not significantly correlate to aGVHD.

However, for renal transplantation it was shown, that the number of determined HLAMatchmaker eplets correlates with the presence of HLA antibodies (R. J. Duquesnoy, "Clinical usefulness of HLAMatchmaker in HLA epitope matching for organ transplantation," Current Opinion in Immunology, vol. 20, no. 5, pp. 594-601, 2008). As shown by (H. G. Otten, J. J. Calis, C. Kesmir, A. D. van Zuilen, and E. Spierings, "Predicted indirectly recognizable HLA epitopes presented by HLA-DR correlate with the de novo development of donor-specic HLA IgG antibodies after kidney transplantation," Human Immunology, vol. 74, no. 3, pp. 290-296, 2013) eplets determined by HLAMatchmaker do not co-localize with PIRCHE peptides determined by the method described herein. This indicates that although HLA matchmaker provides one solution to the problem of predicting HvG immune responses, PIRCHE provides a distinct and complementary approach towards the assessment of this risk.

An additional method of the prior art, HistoCheck, the method evaluated by Spellman and Askar and colleagues, rates the amino acid differences between HLA-allelic products based on the position within the HLA molecule and the functional similarity of amino acids within proteins. The Dissimilarity Scores that were obtained with this ranking system did not predict aGVHD. In the setting of solid organ transplantation, the relevance of the underlying T-cell epitope model was not yet evaluated.

Since the previous attempts with computational methods were unable to predict GVHD, the method of the present invention represents one of the first computer implemented method that provides improved donor selection for HSCT with a reliable and effective pre-transplantation prediction of an unwanted and potentially dangerous immune response. Furthermore, while the previous attempts were undertaken with approaches that mostly asses the structural/functional dissimilarity between HLA molecules (i.e. are based on direct recognition of HLA disparities or the possibility of recognition via antibodies) the present invention preferably is based on predicted indirect recognition of HLA mismatched molecules. The method of the present invention is surprisingly suitable to predict alloreactivity, which was not possible before. The invention therefore is based on the key principle, that the number of predicted indirectly recognizable HLA epitopes (PIRCHES) correlates with the likelihood of an unwanted immune response post-transplantation.

The present invention therefore represents the technical utilisation of the relationship between risk of alloreactivity and increased numbers of mismatched HLA-derived peptides presented by shared-HLA or all MHC class II molecules. These numbers can be determined preferably in silico and can be used as a predictive marker with respect to the development of alloreactivity, for example GVHD or AMR.

The present invention provides a preferably computer implemented method that determines which donor is suited for transplantation when a completely matched donor is not available, without the need for laborious compatibility assays. The present invention for example is applicable to multiple transplant settings, such as stem cells, cord blood cells or solid organ transplantation, amongst others. Essentially any transplantation, in which HLA-matching plays a role in determining alloreactivity or tissue rejection after transplantation, is encompassed by the present invention.

Considering the enormous health cost to patients having suffered from unwanted immune responses after transplantation, methods for the prediction of safely transplantable material are of paramount importance to the medical community. The method as described herein enables reduction of risk upstream of surgery (or treatment), thereby avoiding substantial health and financial cost to patients, medical practitioners and institutions, respectively.

In one embodiment the invention therefore relates to a method for prediction of an immune response against human leukocyte antigens (HLA) associated with, preferably induced by, HLA-mismatches between donor and recipient after transplantation, wherein HLA-typing for the donor and recipient is conducted, at preferably high resolution level with sequence based typing, to determine the mismatches, the number of predicted indirectly recognized HLA epitopes (PIRCHES) is identified using computer-implemented methods by determining the presentation and/or binding of peptides derived from mismatched recipient and/or donor HLA alleles, whereby the number of PIRCHES correlates with the likelihood of said immune response.

In one embodiment the present invention relates to a method as described herein, wherein said transplantation comprises haematopoietic stem-cell transplantation (HSCT).

In one embodiment the present invention relates to a method as described herein, wherein said transplantation comprises cord blood or cord blood cell transplantation.

In one embodiment the present invention relates to a method as described herein, wherein said transplantation comprises kidney transplantation.

The method of the present invention may also be applied for prediction of an unwanted immune response in the context of other medical disorders, such as secondary recurrent miscarriage, antibody formation during pregnancy, or for assessing risk before cornea transplantation.

In one embodiment the present invention relates to a method as described herein, wherein said immune response comprises an unwanted alloreactivity.

In one embodiment the present invention relates to a method as described herein, wherein said immune response comprises a T-cell-mediated response (alloreactivity). In one embodiment the present invention relates to a method as described herein, wherein said immune response leads to acute graft versus host disease (aGVHD), chronic graft versus host disease (cGVHD) or antibody mediated rejection (AMR).

The computer implementation of the invention on the basis of the graph data structure enables an efficient, fast and reliable method for identification of potentially permissible donor material for allogeneic transplantation. The data processed by the software can be handled in a completely or partially automatic manner, thereby enabling in a preferred embodiment an automated computer-implemented method. Data regarding the HLA typing of donor and recipient, in addition to the number of PIRCHES determined for any donor-recipient pair, is stored electronically and maintained in an appropriate graph structure database.

In one embodiment the system may comprise databanks or databases of a cord blood bank, whereby each sample is tested for HLA-type, and the information stored electronically. The system may also comprise a connection between an additional computing device, for example a device of a clinician, transplant centre, or hospital, in which the HLA-type data for the recipient is stored. Through a connection between the two databases, for example over the internet, the method of the invention can be carried out using appropriate software. HLA-types of multiple potential donor samples and the patient may be compared and the number of PIRCHE for any given donor-recipient pair determined. In light of the analysis based on the method described herein a clinically relevant prediction can be made whether any given donor material, for example those samples stored in a cord blood bank or other cell or tissue bank, is suitable for transplantation. The invention also relates to a software suitable for carrying out the method described herein.

According to the present invention, the term "prediction" means a statement about possible events in the future. The term "forecast" may also be used. The "prediction" in the sense of the present invention represents an assessment of the likelihood or risk of an immune response occurring after transplantation. On the basis of the prediction, or risk assessment, valuable information is obtained in advance of a potentially harmful event, which can be used to determine further therapeutic options.

The term "Immune response" in the context of the present invention relates to an immune response as commonly understood by one skilled in the art. An immune response may be understood as a response from a part of the immune system to an antigen that occurs when the antigen is identified as foreign, which preferably subsequently induces the production of antibodies and/or lymphocytes capable of destroying or immobilising the "foreign" antigen or making it harmless. The immune response of the present invention may relate either to a response of the immune system of the recipient against the transplanted material, or an immune response effected by cells of the transplanted cells, tissues, or organs, whereby for example in GVHD T cells of the transplanted material react against and/or attack recipient antigens or tissue. The immune response may be a defence function of the recipient that protects the body against foreign matter, such as foreign tissue, or a reaction of immune cells of the transplanted material against recipient cells or tissue.

The human leukocyte antigen (HLA) system is the major histocompatibility complex (MHC) in humans. The super locus contains a large number of genes related to immune system function in humans. This group of genes resides on chromosome 6, and encodes cell-surface antigenpresenting proteins and has many other functions. The proteins encoded by certain genes are also known as antigens, as a result of their historic discovery as factors in organ transplants. The major HLA antigens are essential elements for immune function. HLAs corresponding to MHC class I (A, B, and C) present peptides from inside the cell (including viral peptides if present). These peptides are produced from digested proteins that are broken down in the proteasomes. In general, these particular peptides are small polymers, about 9 amino acids in length. Foreign antigens attract killer T-cells (also called CD8 positive- or cytotoxic T-cells) that destroy cells. HLAs corresponding to MHC class II (DP, DM, DOA, DOB, DQ, and DR) present antigens from outside of the cell to T-lymphocytes. These particular antigens stimulate the multiplication of T-helper cells, which in turn stimulate antibody-producing B-cells to produce antibodies to that specific antigen.

MHC loci are some of the most genetically variable coding loci in mammals, and the human HLA loci are no exception. Most HLA loci show a dozen or more allele-groups for each locus. Six loci have over 100 alleles that have been detected in the human population. Of these, the most variable are HLA-B and HLA-DRB1.

An allele is a variant of the nucleotide (DNA) sequence at a locus, such that each allele differs from all other alleles by at least one (single nucleotide polymorphism, SNP) position. Most of these changes result in a change in the amino acid sequences that result in slight to major functional differences in the protein.

"HLA" refers to the human leukocyte antigen locus on chromosome 6p21 , consisting of HLA genes (HLA-A, HLA-B, HLA-C, HLA-DRB1 , HLA-DQB1 , etc... ) that are used to determine the degree of matching, for example, between a recipient and a donor of a tissue graft. "HLA allele" means a nucleotide sequence within a locus on one of the two parental chromosomes.

"HLA typing" means the identification of an HLA allele of a given locus (HLA-A, HLA-B, HLA-C, HLA-DRB1, HLA-DQB1, etc...). Samples may be obtained from blood or other body samples from donor and/or recipient, which may subsequently be analysed.

Serotyping (identification of the HLA protein on the surface of cells using antibodies) is the original method of HLA typing and is still used by some centres. Phenotyping relates to the serological approach for typing. This method is limited in its ability to define HLA polymorphism and is associated with a risk of misidentifying the HLA type. Modern molecular methods are more commonly used to genotype an individual. With this strategy, PCR primers specific to a variant region of DNA are used (called PCR SSP). If a product of the right size is found, the assumption is that the HLA allele/ allele group has been identified. PCR-SSO may also be used incorporating probe hybridisation. Reviews of technical approaches towards HLA typing are provided in Erlich H, Tissue Antigens, 2012 Jul;80(1):1-11 and Dunn P, Int J Immunogenet, 2011 Dec;38(6):463-73. Gene sequencing may be applied, and relates to traditional methods, such as Maxam-Gilbert sequencing, Chain-termination methods, advanced methods and de novo sequencing such as shotgun sequencing or bridge PCR, or the so-called "next-generation" methods, such as massively parallel signature sequencing (MPSS), 454 pyrosequencing, Illumina (Solexa) sequencing, SOLiD sequencing, or other similar methods.

With respect to HLA typing, samples obtained from the donor themselves and/or from donor material before, during or after isolation/preparation for transplantation, may be used for HLA-typing and subsequent comparison to the HLA-typing data from the recipient. For example, HLA-typing of the donor themselves, for example by analysing a saliva, blood or other bodily fluid sample for HLA information, may occur, and optionally additionally or alternatively, the material obtained from the donor intended for transplantation (donor material) may be analysed for the same and/or complementary HLA type characteristics during HLA-typing.

An "HLA-mismatch" is defined as the identification of different alleles in donor and recipient, which are present at any given loci.

According to the present invention, the term "two-field specific HLA protein typing" relates to the standardised HLA naming system developed in 2010 by the WHO Committee for Factors of the HLA System. According to the HLA naming system, the nomenclature is as follows: HLA-Gene*Field1:Field2:Field3:Field4-Suffix. Gene relates to the HLA locus (A, B, C, DRB1, DQB1, etc...). Field 1 relates to the allele group. Field 2 relates to the specific HLA protein. Field 3 relates to a synonymous DNA substitution within the coding region. Field 4 is used to show differences in a non-coding regions. The suffix is used to denote particular characteristics in gene expression.

The "two-field" within "two-field specific HLA protein typing" relates to the presence of HLA typing information for Fields 1 and 2 according to the standardized WHO nomenclature used herein. This typing is commonly referred to as "high resolution typing" or "specific HLA protein typing", as the typing provides information on the specific protein sequence relevant for the immune responses dealt with herein.

The term "HLA allele group" typing relates to typing information that is provided for only the HLA gene (locus) and Field1. This typing is commonly referred to as "low resolution" typing. According to the present invention any serotype would be converted to a molecular "low resolution" typing.

The term "intermediate resolution" or "medium resolution" typing relates to some kinds of typing information where the presence of some alleles, or allele strings, have been defined in the patient/donor by the typing method used. The NMDP nomenclature is one method of describing this level of typing. For example A*01:AB relates to alleles 01:01 or 01:02 (01:01/01:02) and the patient/donor is one of these two alleles. The NMDP allele codes are in some cases generic, wherein one code may encompass a number of possible HLA sequences (alleles) at any given HLA locus. Although the donor material may be HLA typed, a number of NMDP codes do not provide information on which specific protein is in fact present at any given HLA locus. This form of typing information is referred to as intermediate resolution typing.

The donor is commonly understood to be an individual or multiple individuals (for example in the case of where multiple samples or preparations, such as cord blood units (CBUs) are required for an effective therapeutically relevant amount of donor material for the transplantation) who provide donor material, or in other words biological material, such as but not limited to cells, tissues, organs, or other bodily parts, fluids and/or preparations, for transplantation in the recipient. References to the donor, or HLA-typing of the donor, may also refer to donor material, or HLA-typing of the donor material, respectively.

The subject recipient of the method is typically a mammal, preferably a human. The recipient is typically a patient suffering from a disorder characterised by the need for transplantation, such as organ failure necessitating a transplant. By the term "organ", it is meant to include any bodily structure or group of cells containing different tissues that are organized to carry out a specific function of the body, for example, the heart, lungs, brain, kidney skin, liver, bone marrow, etc. In one embodiment the graft is an allograft, i.e. the individual and the donor are of the same species. The subject may also suffer from a condition that could be treated by the transplantation of cells, even when the disorder itself is not defined by a lack or loss of function of a particular subset of endogenous cells. Some disorders may be treatable by the transplantation of certain kinds of stem cells, whereby the native or endogenous pool of such cells are not necessarily nonfunctional in the recipient.

The method of the invention is particularly applicable to patients who are about to receive or are predicted to require a cell, tissue or organ transplant, to predict the likelihood of unwanted immune response, such as origin graft damage or rejection, and/or immune origin damage to non-graft tissue. For example, the patient may be expected to receive a transplant in the next one, two, three, four, five, six, or twelve months. Alternatively, the assay is particularly applicable to individuals who have received a transplant to predict the likelihood of immune origin graft damage or rejection, and/or immune origin damage to non-graft tissue. Post-transplant, the method is particularly applicable to patients who show evidence of chronic organ dysfunction (of the graft organ) or possible graft versus host disease (GVHD), particularly chronic GVHD and particularly in cases wherein the graft is a bone marrow transplant.

Transplantation is the moving of cells, tissue or an organ from one body (donor) to another (recipient or patient), or from a donor site to another location on the patient's own body, or from stored donor material to a recipients body, for the purpose of replacing the recipient's damaged or absent organ, or for the purpose of providing stem cells, other cells, tissues or organs capable of providing a therapeutic effect.

Allogeneic transplantation or Allotransplantation is the transplantation of cells, tissues, or organs, to a recipient from a genetically non-identical donor of the same species. The transplant is called an allograft, allogeneic transplant, or homograft. Most human tissue and organ transplants are allografts. Allografts can either be from a living or cadaveric source. Generally, organs that can be transplanted are the heart, kidneys, liver, lungs, pancreas, intestine, and thymus. Tissues include bones, tendons (both referred to as musculoskeletal grafts), cornea, skin, heart valves, nerves and veins.

The invention also encompasses use of the method in the context of screening organs, cells, or tissues produced via regenerative medicine, for example reconstructed donor material that has been constructed ex vivo and is intended for transplantation. Stem cell technologies enable the production of a number of medically relevant cell types or tissues ex vivo. The present invention could therefore also be applied in screening allogeneic material that has been produced by biotechnological and/or tissue engineering methods for its suitability in transplantation.

The invention encompasses the assessment of risk of an immune reaction, preferably a pre-transplantation risk assessment, whereby any given stem cell may be considered as donor material intended for transplantation. For example, hematopoietic stem cell transplantation (HSCT) is the transplantation of multipotent hematopoietic stem cells, usually derived from bone marrow, peripheral blood, or umbilical cord blood. It is a medical procedure common in the fields of hematology and oncology, most often performed for patients with certain cancers of the blood or bone marrow, such as multiple myeloma or leukemia. In these cases, the recipient's immune system is usually destroyed with radiation or chemotherapy before the transplantation. Infection and graft-versus-host disease are major complications of allogenic (also referred to as allogeneic) HSCT.

Stem cells are to be understood as undifferentiated biological cells, that can differentiate into specialized cells and can divide (through mitosis) to produce more stem cells. Highly plastic adult stem cells are routinely used in medical therapies, for example in bone marrow transplantation. Stem cells can now be artificially grown and transformed (differentiated) into specialized cell types with characteristics consistent with cells of various tissues such as muscles or nerves through cell culture. The potential stem cell transplantation may relate to any given stem cell therapy, whereby a number of stem cell therapies exist. Medical researchers anticipate that adult and embryonic stem cells will soon be able to treat cancer, Type 1 diabetes mellitus, Parkinson's disease, Huntington's disease, Celiac disease, cardiac failure, muscle damage and neurological disorders, and many others.

Also known as somatic stem cells and germline stem cells, stem cells can be found in children, as well as adults. Pluripotent adult stem cells are rare and generally small in number but can be found in a number of tissues including umbilical cord blood. Bone marrow has been found to be one of the rich sources of adult stem cells which have been used in treating several conditions including Spinal cord injury, Liver Cirrhosis, Chronic Limb Ischemia and End-stage heart failure. Adult stem cells may be lineage-restricted (multipotent) and are generally referred to by their tissue origin (mesenchymal stem cell, adipose-derived stem cell, endothelial stem cell, dental pulp stem cell, etc.).

Multipotent stem cells are also found in amniotic fluid. These stem cells are very active, expand extensively without feeders and are not tumorigenic. Amniotic stem cells are multipotent and can differentiate in cells of adipogenic, osteogenic, myogenic, endothelial, hepatic and also neuronal lines. It is possible to collect amniotic stem cells for donors or for autologuous use.

Cord blood-derived multipotent stem cells display embryonic and hematopoietic characteristics. Phenotypic characterization demonstrates that (CB-SCs) display embryonic cell markers (e.g., transcription factors OCT-4 and Nanog, stage-specific embryonic antigen (SSEA)-3, and SSEA-4) and leukocyte common antigen CD45, but that they can be negative for blood cell lineage markers. Additionally, CB-SCs display very low immunogenicity as indicated by expression of a very low level of major histocompatibility complex (MHC) antigens and failure to stimulate the proliferation of allogeneic lymphocytes.

HSC are typically available from bone marrow, Peripheral blood stem cells, Amniotic fluid, or Umbilical cord blood. In the case of a bone marrow transplant, the HSC are removed from a large bone of the donor, typically the pelvis, through a large needle that reaches the center of the bone. The technique is referred to as a bone marrow harvest and is performed under gen-eral anesthesia. Peripheral blood stem cells are a common source of stem cells for allogeneic HSCT. They can be collected from the blood through a process known as apheresis. The donor's blood is withdrawn through a sterile needle in one arm and passed through a machine that removes white blood cells. The red blood cells may be returned to the donor. The peripheral stem cell yield may be boosted with daily subcutaneous injections of Granulocyte-colony stimulating factor, serving to mobilize stem cells from the donor's bone marrow into the peripheral circulation.

It is also possible to extract hematopoietic stem cells from amniotic fluid. Umbilical cord blood is obtained from an infant's Umbilical Cord and Placenta after birth. Cord blood has a higher concentration of HSC than is normally found in adult blood. However, the small quantity of blood obtained from an Umbilical Cord (typically about 50 ml.) makes it more suitable for transplantation into small children than into adults. Multiple units could however be used. Newer techniques using ex-vivo expansion of cord blood units or the use of two cord blood units from different donors allow cord blood transplants to be used in adults. Cord blood can be harvested from the umbilical cord of a child being born.

Unlike other organs, bone marrow cells can be frozen (cryopreserved) for prolonged periods without damaging too many cells. This is a necessity with autologous HSC because the cells are generally harvested from the recipient months in advance of the transplant treatment. In the case of allogeneic transplants, fresh HSC are preferred in order to avoid cell loss that might occur during the freezing and thawing process. Allogeneic cord blood is typically stored frozen at a cord blood bank because it is only obtainable at the time of childbirth. To cryopre-serve HSC, a preservative, DMSO, must be added, and the cells must be cooled very slowly in a controlledrate freezer to prevent osmotic cellular injury during ice crystal formation. HSC may be stored for years in a cryofreezer, which typically uses liquid nitrogen. In light of this, the invention may relate to typing and risk assessment of donor material already stored as described herein, before being considered for transplantation.

The invention encompasses the assessment of risk of an immune reaction, preferably a pre-transplantation risk assessment, whereby any given organ or tissue may be considered as donor material intended for transplantation. For example, kidney transplantation or renal transplantation is the organ transplant of a kidney into a patient, for example with end-stage renal disease. Kidney transplantation is typically classified as deceased-donor (formerly known as cadaveric) or living-donor transplantation depending on the source of the donor organ. Living-donor renal transplants are further characterized as genetically related (living-related) or non-related (livingunrelated) transplants, depending on whether a biological relationship exists between the donor and recipient.

Alloreactivity is defined as the reaction of a lymphocyte or antibody with an alloantigen, which may be understood as an antigen from foreign material. Alloantigen recognition may occur via direct or indirect alloantigen recognition, by which T cells may recognize alloantigens and potentially lead to transplant rejection after an organ transplant.

Graft-versus-host disease (GVHD) is a relatively common complication following an allogeneic cell, tissue or organ transplant. It is commonly associated with stem cell or bone marrow transplant but the term also applies to other forms of tissue graft or organ transplant. Immune cells (typically white blood cells) in the tissue (the graft) recognize the recipient (the host) as "foreign". The transplanted immune cells then attack the host's body cells. GVHD can also occur after a blood transfusion if the blood products used have not been irradiated.

Proteasomes are protein complexes inside all eukaryotes and archaea, and in some bacteria. In eukaryotes, they are located in the nucleus and the cytoplasm. The main function of the proteasome is to degrade unneeded or damaged proteins by proteolysis, a chemical reaction that breaks peptide bonds. Most antigenic peptides presented by MHC class I molecules result from the degradation of intracellular proteins by the proteasome. Proteasome degradation of mismatched HLA antigens can be predicted by computational tools as described herein.

### FIGURES

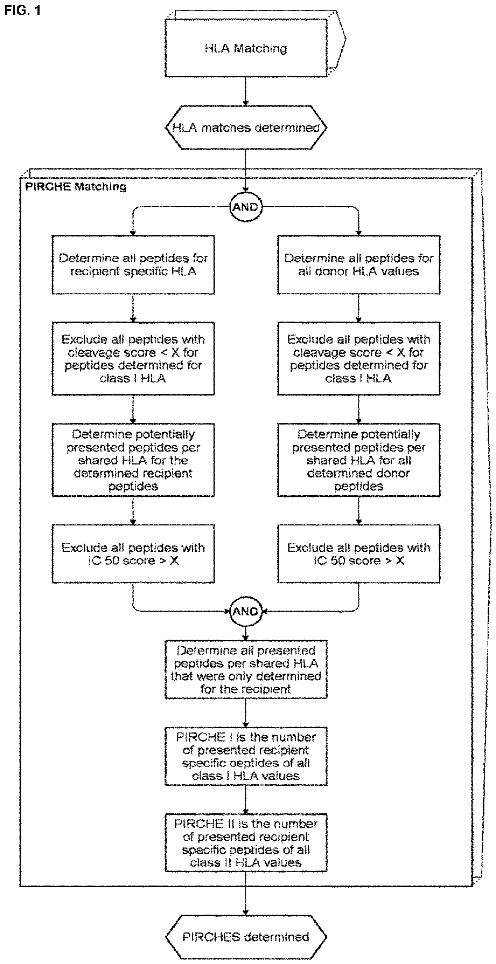

Fig 1: Schematic representation of peptide cleavage and binding simulation
Fig 2: Schematic of Graph data structure. From a functional perspective the core data structure for the algorithm consists of HLA Values, Peptides and relationships between those entities.
Fig 3: Example of a graph data structure comprises nodes for peptide and HLA value entities, and "has" or "presents" relationships between said entities.
Fig 4: Schematic representation of the combined HLA matching and PIRCHE matching software modules, preferably via a service integration manager (presentation layer).
Fig 5: provides a more detailed overview of a potential system architecture as an example.

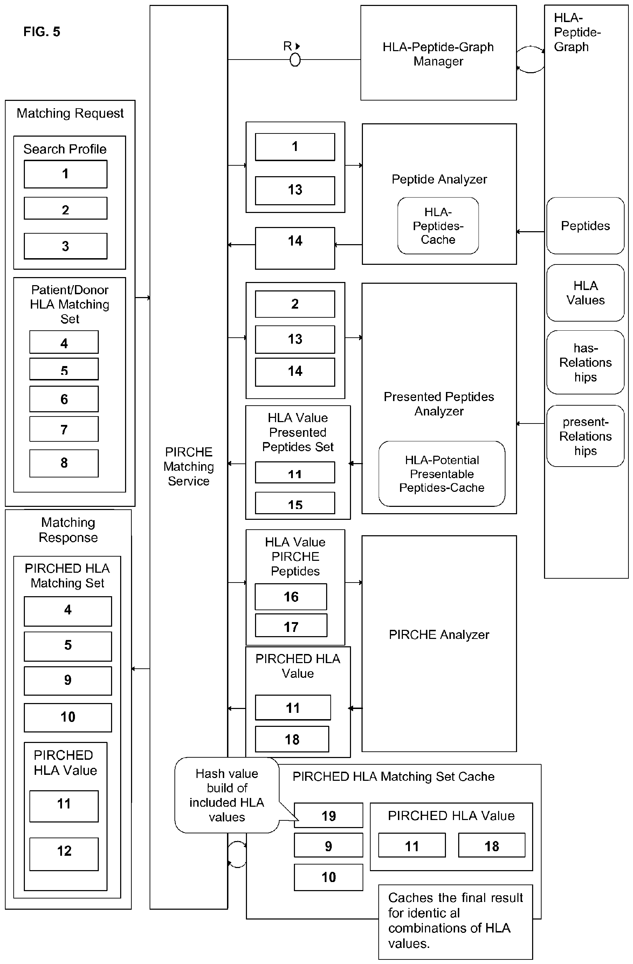

Reference signs for Figure 5:
1. Cleavage Threshold
2. IC 50 Score Threshold
3. Matching Direction
4. Patient ID
5. Donor ID
6. Mismatched Patient HLA
7. Mismatched Donor HLA
8. Shared HLA
9. PIRCHE I
10. PIRCHE II
11. HLA Value
12. PIRCHE Value
13. HLA Value Set
14. HLA Value Set Peptides
15. HLA Value Presented Peptides
16. Patient Presented Peptides
17. Donor Presented Peptides
18. PIRCHE Value
19. Patient/Donor HLA Value Set ID

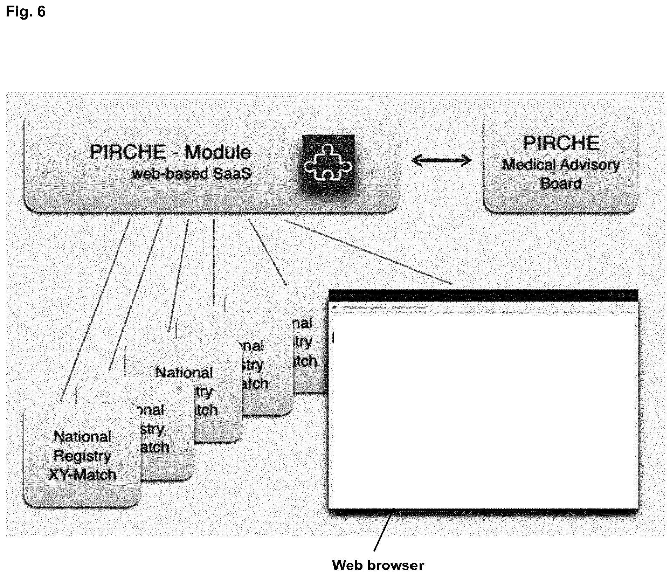

Fig. 6: provides a schematic view of the software implementation of the present invention, in particular the PIRCHE module linked with the web based SaaS, in combination with a medical advisory board.

### EXAMPLES

### Relevant information for the graph data structure

The graph data structure of the present invention comprises essentially all MHC class I HLA alleles and lists all possible peptides for each allele with a score value for the proteasome cleavage prediction. In one embodiment the predictions are based on the NetChop C term 3.0 prediction method. The information may comprise:
- HLA-ID: ID of an HLA allele as defined in the IMGT/HLA database (accession number)
   ∘ Example: HLA00005
- Peptide: Peptide with a length of 5 to 15, preferably 7 to 11 or more preferably 9 amino acids. The most common length of peptides presented by MHC class I is 7-11 amino acids.
   ∘ Example: MAVMAPRTL (SEQ ID NO 1)
- Cleavage Score: Value ranging from 0.00-1.00 expressing the cleavage prediction for the peptide
   ∘ Example score value for MAVMAPRTL (SEQ ID NO 1): 0.583079
   ∘ A higher value indicates a higher cleavage probability.

The score represents a property of the relationship between a peptide and the HLA allele from which it is derived.

The data structure also contains relationships from all peptides potentially presented by any given MHC class I or class II HLA allele, in addition to the originating HLA allele that may lead to the peptide after cleavage. For each potentially presented peptide an IC 50 score representing the binding affinity is listed (preferably generated by NetMHCpan), which is considered a property of the relationship between a peptide and its likelihood of being presented by any given HLA allele.
- IC 50 Score: Value ranging from 0.00-2000.00 expressing the binding affinity of the peptide
   ∘ Example score value for MMVLQVSAA (SEQ ID NO 2): 285.57
   ∘ A lower value indicates a higher binding affinity.

For HLA class II molecules the following information may be present:
- Peptide: Peptide with a length of preferably 15 amino acids. The antigens presented by MHC class II molecules are typically longer than those presented by class I. Therefore, for the binding prediction a length of 15 amino acids is used.
   ∘ Example: RAYLEGTCVDGLRRY (SEQ ID NO 3)
- Score: Value ranging from 0.00-2500.00 expressing the binding affinity of the peptide
   ∘ Example score value for RAYLEGTCVDGLRRY (SEQ ID NO 3): 476.80
   ∘ A lower value indicates a higher binding affinity.

### Outline 1:

The method as described in the following is designed to work in Graft vs. Host (GvH) direction.

Determining PIRCHE I for MHC class I HLA:
Determine all recipient specific peptides that may potentially be presented by shared HLA:
Find the mismatched HLA allele between recipient and donor before initiating method, or as initial step in method, comprising for example:
a. HLA allele matching: shared HLA
b. HLA allele mismatch: mismatched HLA
   1. For the mismatched recipient HLA allele:
      a. Determine all peptides that have a cleavage prediction > 0.5 (the threshold might be adjusted)
   2. For every shared class I HLA allele of the recipient:
      a. Check for all peptides determined in 1.a if there is a relationship with an IC50 score < 500 (the threshold might be adjusted)

Determine peptides for all donor HLA values that may potentially be presented by shared HLA:
3. For every donor HLA allele:
   a. Determine all peptides that have a cleavage prediction > 0.5 (the threshold might be adjusted)
4. For every shared class I HLA allele of the donor:
   a. Check for all peptides determined in 3.a if there is a relationship with an IC50 score < 500 (the threshold might be adjusted)

Compare determined peptides of donor and recipient for each shared HLA:
5. and 6. For every shared HLA allele:
a. Determine all peptides presented by the recipient HLA molecule that were not determined for the corresponding donor HLA molecule

Determining PIRCHE II for MHC class II HLA:
- basic method is the same as for class I HLA
- process all peptides independent of cleavage score
- use 15mer peptides for binding prediction

Preferred embodiments:
- molecular high resolution allele codes are preferably supported (e.g. A*02:01)
- loci A*, B*, C*, DRB1* and DQB1* are preferably supported

### Practical Examples:

**Table 1.: Example of HLA-Mismatch**

| Name | HLA-A | -B | -C | -DRB1 | -DQB1 |
|---|---|---|---|---|---|
| Recipient | *01:01 | *07:02 | *07:01 | *03:01 | *02:01 |
| | *11:01 | *08:01 | *07:04 | *11:01 | *03:01 |
| Expected donor 1 | *01:01 | *07:02 | *07:01 | *03:01 | *02:01 |
| (BMDW) | *11:01 | *08:01 | *07:02 | *11:01 | *03:01 |

### Example Outline:

1. All recipient peptides of mismatched HLA (C*07:04) have to be determined (with certain thresholds).
2. For each shared HLA (A*01 :01, A*11:01,...) it has to be checked which of the peptides determined in 1 it can potentially present (with certain thresholds).
3. Determine all peptides of all HLA values of the donor.
4. For each shared HLA (A*01:01, A*11:01,...) it has to be checked which of the peptides determined in 3 it can potentially present (with certain thresholds).
5. For each shared Class I HLA molecule: Any peptide determined in 2 that is not included in the set of peptides in 4 is a PIRCHE I.
6. For each shared Class II HLA molecule: Any peptide determined in 2 that is not included in the set of peptides in 4 is a PIRCHE II.

This means one peptide could theoretically lead to more than 1 PIRCHE as it could be presented by more than one HLA molecule.

**Table 2.: The table below shows examples considering only loci C* and DRB1* and is based on artificial peptide data to illustrate the concept.**

| **Example 1** | | **has Peptides** | **presents Peptides** | **presented Peptides** | **presented recipient specific peptides** | **PIRCHE I** | **PIRRCHE II** |
|---|---|---|---|---|---|---|---|
| **Recipient** | C*07:01 | A.B | A,G,X | A,G | | | |
| | C*07:04 | A,C,J | A,F,C | /na | /na | | |
| | DRB1*02:01 | D,E | A,B | A,B | | | |
| | DRB1*03:01 | F,G | C,Y | C | C | | 1 |
| **Donor** | C*07:01 | A,B | A,G,X | A,G | | | |
| | C*07:02 | A,H | A,C | /na | /na | | |
| | DR81*02:01 | D,E | A,B | A,B | | | |
| | DRB1*03:01 | F,G | C,Y | | | | |
| | | | | | **PIRCHE Score** | | **1** |
| | | | | | | | |
| | | | | | | | |

| **Example 2** | | **has Peptides** | **presents Peptides** | **presented Peptides** | **presented recipient specific peptides** | **PIRCHE I** | **PIRCHE II** |
|---|---|---|---|---|---|---|---|
| **Recipient** | C*07:01 | A,B | A,G,X | A,G | | | |
| | C*07:04 | A,C,J | A,F,C | | /na | | |
| | DRB1*02:01 | D,E | A,B | A,B | | | |
| | DRB1*03:01 | F,G | C,Y | | C | | 0 |
| **Donor** | C*07:01 | A,B | A,G,X | | | | |
| | C*07:02 | A,H,C | A,C | | /na | | |
| | DRB1*02:01 | D,E | A,B | A,B | | | |
| | DRB1*03:01 | F,G | C,Y | | C | | |
| | | | | | **PIRCHE Score** | | **0** |

### Example 1:

1. All recipient peptides of mismatched HLA (C*07:04) have to be determined (with certain thresholds).
   - The recipient "has" the peptides A, B, C, J, D, E, F, G.
2. For each shared HLA it has to be checked which of the peptides determined in 1 it can potentially present (with certain thresholds).
   - The HLA molecule for DRB1*03:01 can potentially present the peptides C and Y. As the donor does not "have" Y the HLA molecule can actually only present C. For the mismatched HLA molecule the presented peptides are not relevant.
3. Determine all peptides of all HLA values of the donor.
   - The donor "has" the peptides A, B, H, D, E, F, G.
4. For each shared HLA it has to be checked which of the peptides determined in 3 it can potentially present (with certain thresholds).
   - The HLA molecule for DRB1*03:01 can potentially present the peptides C and Y. As the donor does not "have" C or Y the HLA molecule can actually not present any of the peptides. For the mismatched HLA molecule the presented peptides are not relevant.
5. For each shared Class I HLA molecule: Any peptide determined in 2 that is not included in the set of peptides in 4 is a PIRCHE I.
6. For each shared Class II HLA molecule: Any peptide determined in 2 that is not included in the set of peptides in 4 is a PIRCHE II.
   - Comparing the presented peptides for DRB1 *03:01 between recipient and donor results in the peptide C that is only presented at the recipient. As DRB1*03:01 is a class II allele this is a PIRCHE II.

### Example 2:

- In this example it is assumed that the allele C*07:02 may have the peptides A, H and C, i.e. the donor does now "have" the peptide "C".
- DRB1*03:01 HLA molecules can potentially present C peptides.
- Comparing the presented peptides for DRB1 *03:01 between recipient and donor results in all the peptides of the recipient being presented by the donor, too. Therefore, there is no PIRCHE for DRB1*03:01.

### Outline 2:

In the following, the algorithm is described in the host versus graft (HvG) direction. This direction will be applied when the patient's immune system is assumed or expected to react against foreign tissue (e.g. solid organs, blood cells).

Basically, the PIRCHE value for antibody recognition (HvG) is the number the peptides obtained from the donor HLA molecules that do not match any HLA peptide derived from the patient (recipient) and that are likely to be presented by the patient's MHC class II HLA molecules.

Preferably, the PIRCHE value for SOT (or other HvG analysis) is based on peptides "cleaved" in silico from mismatched donor HLA molecules, either with a cleavage prediction or preferably without the use of proteasome cleavage prediction. For example, when no cleavage likelihood prediction is used, an in silico cleavage of a peptide (preferably but not limited to a 15mer amino acid peptide) is carried out at an interval of 1 amino acid. In other words, at every amino acid of the mismatched HLA allele a peptide is "cleaved" and assessed for presentation in a MHC class II molecule.
1. Given the patient's high resolution HLA typing, get all peptides (preferably 15mers) that are likely to be cleaved, if no appropriate score prediction method is available assume p=1.0 for each peptide.
2. Given the donor's high resolution HLA values that do not match with the patient (mismatched HLA alleles), get all peptides (preferably 15mers) that are likely to be cleaved, if no appropriate score prediction method is available assume p=1.0 for each peptide
3. Determine all donor's peptides gathered in (2) that were not found in (1)
4. PIRCHEs are all peptides of (3) that have a high binding affinity (a lower IC 50 score than a parameterized threshold) on the patient's MHC class II HLA molecules

### Example 1:

The patient typing (all HLA values) can be cleaved into peptides A, B, C, D, E, F, G. The donor typing can be cleaved into the peptides A, B, D, E, F, G, H. The patient has a DRB1*03:01 and a DRB1 *07:01. Assuming the DRB1 *03:01 has low binding affinities for the unknown peptide, it will not present any PIRCHE. Assuming the DRB1*07:01 has a high binding affinity for peptide H, it presents H as a PIRCHE. Therefore, the PIRCHE value for the given donor is 1.

### Data Structure

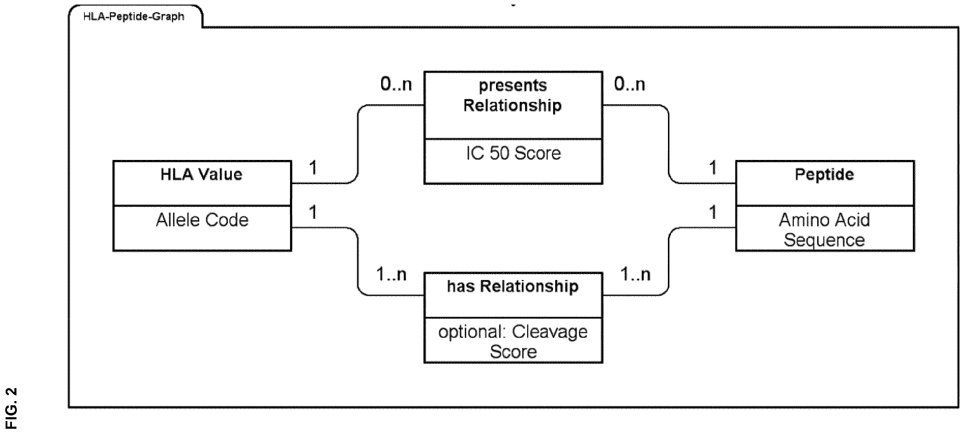

From a functional perspective the core graph data structure for the algorithm consists of HLA Values, Peptides and relationships between those entities (see Fig 2).

An HLA Value "has" a number of Peptides, meaning the proteasome cleavage of the amino acid sequence of the HLA Value may potentially result in the related Peptides. The likelihood of this cleavage is expressed by the Cleavage Score for class I HLA. For class II HLA cleavage this values is preferably not maintained, as cleavage patterns may not be well defined yet.

Furthermore, an HLA value may "present" a number of Peptides, meaning the Peptide may be bound to and presented by the HLA molecule on the cell surface. The IC 50 score expresses the likelihood of the actual binding of a Peptide to an HLA molecule. The IC 50 score is defined for relationships to class I and class II HLA Values.

Preferably, Peptides with a sequence of 9 amino acids are used as potentially presented Peptides by class I HLA and Peptides with a sequence of 15 amino acids are used as potentially presented Peptides by class II HLA. However, other Peptide length may be included in subsequent releases.

The graph data structure provides HLA Value entities, Peptide entities and relationships between those entities. The structure comprises all relevant peptides and the corresponding cleavage scores for all relevant HLA value entities, including Class I and Class II Binding Predictions.

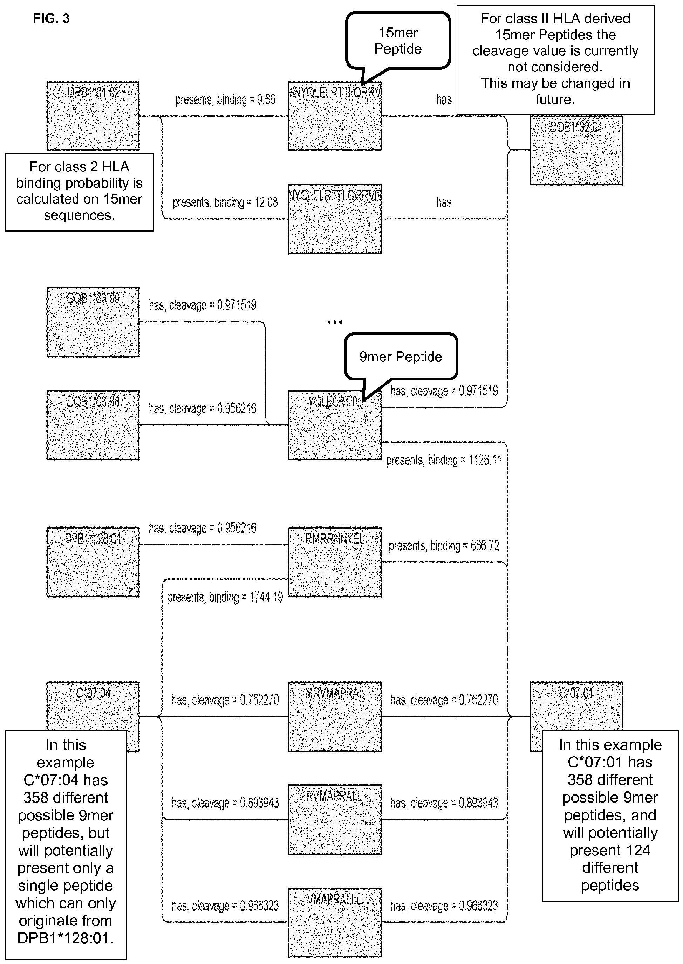

The graphic in Fig. 3 depicts an example segment of this data graph. This data structure can ideally be stored in a graph database in the form shown in the graphics above. Alternatively, suitable storage concepts include storage in key value stores and storage in relational data base tables.

As shown in the example of Fig. 3, the abstraction of the real world entities in a graph structured data model is ideally reflecting the biological background. Therefore, for the actual storage of the data in the system a graph database is preferred. Entities are stored as unique nodes, thereby enabling efficient processing of data.

Key value stores offer performant look up of data while not being optimal in terms of size as entities may have to be stored redundantly. This concept represents one embodiment of the invention, in particular if performance becomes a problem and is ideal for caching.

Relational database management systems are optimized for aggregating data and also offer ideal storage but will need frequent join operations on large data tables. This might have a negative impact on performance.

A graph data base is optimized for highly connected data and allows a high performance access to relationships in combination with an ideal minimized storage size. Furthermore, when the biological background factors to be considered in the matching method described herein are extended and the domain model becomes more complex, the graph structure will allow ideal integration of such extensions.

### The following scales should be considered:

Peptides: ∼3.000.000 (∼1.832.000 9mer peptides + 15mer peptides)
HLA Values: ∼ 10.000
Relationships: ∼ 10.000*1000

It is sufficient, and preferred, to only include those Peptides in the graph structure that can potentially be presented by at least one HLA molecule.

### System Architecture

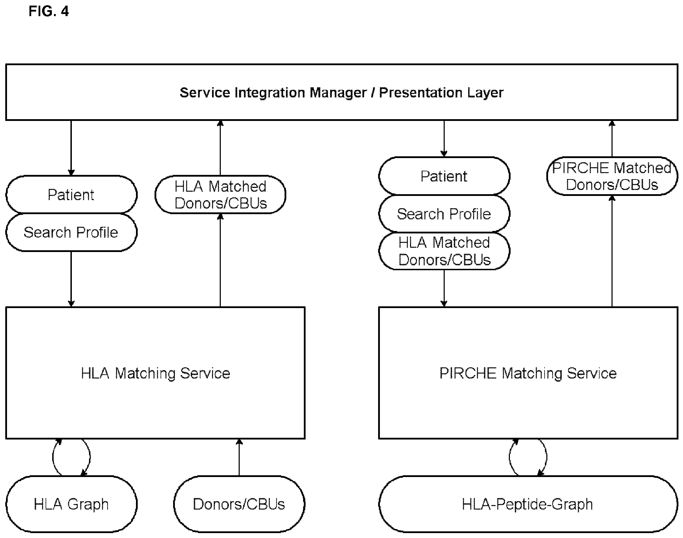

Fig 4 discloses a schematic representation of the combined HLA matching and PIRCHE matching software modules, preferably via a service integration manager (presentation layer).

The PIRCHE Matching module is designed as a service component that can be integrated into other applications. This allows offering the PIRCHE Matching Service as a standalone service as well as integration into existing applications, like HLA Matching systems.

As the PIRCHE Matching Service needs to be provided with identified HLA matches and mismatches between patients and donors integration into an existing HLA Matching system is an ideal combination.

As the PIRCHE Matching Service prefers high resolution typed HLA allele values and these are often not available for many donors a high resolution HLA genotype estimation service is another service integration that leads to a more sophisticated solution.

The combination of HLA Matching Services, high resolution HLA genotype estimation services and the PIRCHE Matching Service offers new possibilities like the applicability of the solution to large registries of not high resolution typed donors, the integration of the results of all services into a scoring system to rank donors according to HLA matching, high resolution HLA estimation, PIRCHEs and other factors like cell count, blood group, infectious diseases markers, gender and age in combination. For a search coordinator or physician this will allow the identification of better HSCT donors in shorter time leading to better treatment outcomes.

The PIRCHE Matching Service accepts molecular high resolution patient HLA data, a search profile and a list of molecular high resolution HLA values, e.g. of matched donors or CBUs, i.e. the matching and mismatching HLA values have to be identified.

Based on the HLA-Peptide-Graph the HLA-Peptide-Matching algorithm analyses the PIRCHE I and PIRCHE II values of each donor/CBU and returns the given Donors/CBUs list enriched with the PIRCHE information to the service client.

To integrate the service into another system it is preferred to have a superior Service Integration Manager Component that is the service client and orchestrates it with other services like the HLA Matching Service and the high resolution HLA genotype estimation services to provide a comprehensive solution.

Fig 5 provides a more detailed overview of potential system architecture as an example.

The graphic in Fig 5 describes the PIRCHE Matching Service Component in more detail. The Matching Service consists of the Main Service Layer and several sub components, the most important being the Peptide Analyzer, the Presented Peptides Analyzer, the PIRCHE Analyzer and the HLA-Peptide-Graph Manager. Furthermore, it consists of the HLA-Peptide-Graph store and a number of caches to improve performance.

The PIRCHE Matching Service is called by the client with a Matching Request data structure and provides a Matching Response data structure as the result.

The Matching Request data structure consists of a Search Profile and set of Patient/Donor HLA Matching Sets. The Search Profile specifies the Cleavage Threshold, the IC 50 Score Threshold for the peptide binding affinity and the requested Matching Direction (Graft vs. Host or Host vs. Graft).

A Patient/Donor HLA Matching Set contains the HLA Matching result of a patient and a donor. I.e. it contains the shared and the mismatched HLA values between patient and donor. As the mismatches are dependent on the patient donor combination this has to be provided for each donor to be analyzed. In addition, it contains the ID of the patient and the ID of the donor for identification purposes.

The Matching Response contains PIRCHED HLA Matching Sets. Each set comprises a set of PIRCHED HLA Values, each consisting of the HLA Value and the corresponding number of PIRCHE I or PIRCHE II that was determined. Additionally, the PIRCHED HLA Matching Sets contain the total number of PIRCHE I or PIRCHE II for the considered HLA loci and the ID of the patient as well as the ID of the donor for identification purposes.

Internally the system may be made up of the following sub components:
- The HLA-Peptide-Graph Manager is responsible for creating and updating the HLA-Peptide-Graph.
- The Peptide Analyzer is determining the HLA derived peptides based on the HLA-Peptide-Graph for a given set of HLA values with an optional cleavage threshold.
- The Presented Peptides Analyzer is determining which peptides of a given set are potentially being presented on a set of given HLA molecules with a given IC 50 Score Threshold based on the HLA-Peptide-Graph. The result consists of a set of potentially presented peptides for every given HLA value.
- The PIRCHE Analyzer takes HLA values and corresponding sets of patient and donor presented peptides and determines for every HLA value the number of PIRCHE I or PIRCHE II.

### Main Use Case of combined HLA match/PIRCHE system

1. User logs into HLA Match/PIRCHE system
2. User creates a new patient
3. User enters patient HLA
4. User navigates to Search
5. User configures search profile and starts search
6. System performs search with search profile settings
7. System shows Search Result Screen
8. User can switch between CBU result list and donor result list
9. User exports donors as csv file (optional)

In a preferred embodiment it is possible to display a donor search result and to switch between CBU and donor search result.

### Donor Type Search

The solution will comprise a donor genotype database on which the PIRCHE Matching Service can be executed for a given patient to determine a list of potential donor types that have a genotype with permissible mismatches.

| | | | | | | | | | | | |
|---|---|---|---|---|---|---|---|---|---|---|---|
| Donor Type ID | Score | HvG | GvH | A* | B* | C* | DRB1* | DQB1* | PIRCHE I | PIRCHE II | Freque ncy |

The columns of the Donor Search Result Screen are:
- Donor Type ID
- Score (For first release: PIRCHE I + PIRCHE II, may be extended later)
- HvG (Host vs Graft mismatches)
- GvH (Graft vs Host mismatches)
- A*
- B*
- C*
- DRB1*
- DQB1*
- PIRCHE I
- PIRCHE II
- Frequency (Frequency of this donor genotype based on the haplotype frequencies used to create the donor genotype database)
- (Ethnicities of Donor Types will be added in subsequent releases)

The donor genotype database will allow searching for donor genotypes with permissible mismatches. A typical search in an existing donor registry of real donors might lead to not realizing a 10/10 matched donor genotype at all, for example if such a donor is not present in the searched registry. By searching the donor genotype database with HLA and PIRCHE Matching, genotype search templates may be determined that allow to steer further searches in the right direction, e.g. by identifying donor types that should be frequent in certain registries, e.g. because of gender, sex, ethnicity, etc...

The PIRCHE Matching Module can also be used to match real CBUs and donors. The user can switch between the result lists of the CBU inventory, the real donor inventory and the donor genotype database.

### HLA Graph-database improved PIRCHE speed

For validation purposes an artificial test file of patients and accompanied donors was generated. This file contains 26.000 pairs of theoretical patients & donors of hematopoietic stem cells with 1 mismatch in a 10 out of 10 HLA typing as well as PIRCHE I+II values generated by each pair.

The construction principle is as follows. Patient and donor have 1 serological mismatch in HLA-A locus. This mismatch is fixed in position and the kind of allele throughout all following pairs. The following patient & donor pairs vary by permutation of all existing alleles of the HLA loci HLA-A, HLA-B, HLA-C, HLA-DRB1, and HLA-DQB1 simultaneously at the site of the patient and donor. The permutation of the matched or shared alleles leads to different PIRCHE values even for this single fix mismatch in each of the pairs.

This file was used to validate the correctness of the algorithm transfer from an earlier Perl Script implementation into the new HLA Graph database structure. Correctness was given when all PIRCHE values where the same in the original Perl Script as well as in the Graph database.

The original implementation needed 11hours to perform the calculation for all 26,000 pairs. The implementation described herein using the graph data structure took only 10 seconds to perform all 26,000 PIRCHE calculations.

The speed improvement together with the web browser based user interface now allows an interactive use of the algorithm. Now clinical working situations of 1 patient with 10 to 100 potential donors run in parts of a second. The patient can be tested very quickly with different pools of potential donors. e.g. coming from different registries or different countries. No waiting necessary, answers come immediately and are available everywhere where a web browser provides access to the internet.

The graph data structure therefore provides not only improved speed in computing. Due to the biological context of the data structure, the biological samples represented in the data (not directly, but assuming the data corresponds indirectly to patient samples) and ultimately the improved speed of identifying permissible mismatched HLA donor material, the invention provides unexpected technical effects and benefits. Until present, earlier methods of matching and selection of permissible mismatched HLA donor material were slow and limited by both the biological approaches applied (i. e. searching only for 10/10 matches) and the relatively inefficient computing approaches applied previously.

The present invention is therefore defined by a combination of novel features regarding the biological context and mechanism employed to identify permissible mismatches (indirect presentation vs direct presentation), in addition to the novel data structure employed for speedier processing. The combination of these features leads to unexpected synergy, resulting in much faster processing times and therefore entirely novel approaches towards interrogating multiple stem cell or donor tissue databases and registers from around the world in search efforts to find permissible donor material.

### Software implementation

The system described herein was incorporated into an intuitive visual browser interphase enabling access to the underlying HLA Graph-database. The visual interface also lead to improved PIRCHE speed and enables a more interactive working style during donor material search. Previous attempts at HLA matching and similar search engines are severely limited by their slow processing times. According to som early systems, the results to particular inquiries would be provided first one or more days after requests had been sent.

The graph database structure enables a reduction in test file runtime from 11h down to 10sec (≈26.000 patient / donor pairs).

The software implantation of the system incorporates a Software as a Service (SaaS) Json - REST interface that allows PIRCHE integration into any given existing donor selection system already existing. The invention (data structure and interrogation thereof) may therefore itself be represented as a module that is capable of integration with other HLA matching services.

### Example 2:

Further testing and analysis of the data structure, method and software implementation as described in Example 1 has been conducted by the inventors and the following advantages have been obtained and are illustrated by the following:
The improved performance achieved through the data structure of the present invention allows searching donor profiles (donor/patient matches) for HSCT in order to:
- Define permissive mismatches that are asynchronous at the time of allocation, and/or
- Match the patient against a panel of virtual 9/10 donors to find "low PIRCHE donors", or virtual desired HLA profiles that are associated with low risk due to their low PIRCHE number, that one should identify virtually and then search for in existing donor databases in order to reduce risk of an unwanted transplantation immune reaction.

The improved performance due to the graph data structure described herein allows usage in real-time for organ allocation (i.e. for deceased donors). PIRCHE can now effectively be taken into account when deciding for a deceased donor, which patient benefits the most/has the lowest risk of developing donor specific antibodies, in advance of delivering administering the transplantation material.

The high performance of the system described herein allows using PIRCHE with low-resolution, serologic or ambiguously typed patients/donors. Methods can be employed in which all potential sequence-specific HLA profiles are generated virtually for donors who have only been typed at low or medium resolution. These potential HLA profiles can then be assessed for PIRCHES, thereby identifying which low or medium resolution profile is associated with a likelihood of exhibiting low PIRCHE numbers.

The high performance of the present invention further enables significant improvements in reduced time of processing.

For example, ambiguous typings increase the runtime quadratically → O(i*j) = O(i²)
(Likely patient typings = i; Likely donor typings = j)

The computational complexity of the method can be represented as follows:
- (n=number of known alleles)
- (r=number of patients)
- (d=number of donors per patient)
- (k=number of loci taken into account)
- naive approach: O(r*d*n²*k²)
- Approach based on the data structure and methods described herein: O(r*d*k)

The PIRCHE data structure described herein allows implementation with runtime independent of number of alleles. This is a huge benefit, as IMGT/HLA database grows exponentially.

This is illustrated when considering space complexity:
- Naive approach: O(n²)
- PIRCHE AG: O(n)
- PIRCHE data structure size is only linearly dependent on number of alleles

Space complexity of naive approach directly affects computational complexity.

Naive implementation of the PIRCHE method dealt only with ∼5500 4-digit alleles:
- Took 11 hours for 26k pairs
- Current implementation of IMGT HLA database contains ∼10800 4-digit alleles (HLA-A, B, C, -DRB1, -DQB1, -DPB1)
- Number of known alleles increased by 60% within last 3 years, and will increase further
- With respect to complexity analysis, a doubled amount of data in a database means 4 times the runtime → 26k pairs with current database might take ∼44 hours with naive approach. The data structure of the present invention leads to significant improvements.

As a further example, organ transplantation studies identify further advantages. The present invention allows prospective real-time usage as required by deceased donor allocation. For example, deceased donor allocation in Eurotransplant considers that ischemia time should be as low as possible to avoid organ damage, whereby every hour post-obtaining the transplantation material is extremely important. The waiting list size was 8,000 transplantable patients in 2015. Even when restricted to the top 100 patients, carrying out the method as described herein would require 2,300 matches (single donor with low resolution typings).
The naive approach would require greater than 1h for the computational analysis, whereby with the graph data structure described herein the analysis is conducted in 21 seconds (assuming single CPU).

Additional benefits are obtained on the computer processing itself by using the data structure of the present invention. Implementation of the present invention allows linear scaling with computation cores (e.g. CPU cores). Even without scaling hardware, the given approach achieves runtimes capable of serving today's use cases.

A further example relates to crossover living donors. Complex allocation scenarios may arise in the future due to low numbers of deceased donors and ever increasing number of patients who would be suitable as recipients of transplantations. Complex crossover scenarios may occur where for example a Living donor 1 for Patient 1 donates a kidney for Patient 2, whereas Living donor 2 donates a kidney for Patient 1. This procedure can be arbitrarily complex, i. e. in the case of a Swiss 3 pair crossover transplantation.

Assuming only 100 patients on a crossover waiting list, finding an optimal solution (independent of the complexity of the solution) already results in 10,000 match pairs to be analysed. Assuming low resolution typings, this leads to up to 230,000 PIRCHE matches to be assessed. Even with parallelization the naive approach is exhausted by this task and is unsuitable for practical application.

A further example relates to risk profiles for solid organ transplants. The performance boost obtained by the present application allows usage of PIRCHE for creating a "risk profile". The user can run PIRCHE against the most likely donors in a local population (using either population allele frequencies (i.e. low res patient vs millions of high res donors) or real donor panels (i.e. low res patient vs thousands of low res donors)). In order to comply with the immense variability of the HLA genes, this requires millions of matches to be assessed. Individual results can be classified and combined with virtual donor frequencies to obtain a probability as to whether the patient can find a low PIRCHE donor suitable for the transplantation. In any particular case, if a score implies a low chance of finding a low PIRCHE donor in the population, and an actually offered organ (from a deceased donor) has a low PIRCHE score, the organ should then be transplanted, as it is unlikely that a donor with an equally low PIRCHE score will appear randomly in the near future. The performance of the data structure described herein therefore enables population frequency assessment in order to evaluate which patients require deceased donor transplants.

## Claims

1. A computer implemented method for determining the numbers of predicted indirectly recognized HLA epitopes (PIRCHES) between one or more donors and one or more recipients of solid organ transplantation material,
wherein said PIRCHES are donor-specific HLA-derived peptides from a mismatched donor-HLA allele and are predicted to be presented by any MHC class II HLA molecule, wherein said method comprises
a) Identification of peptides from mismatched HLA molecules of the donor;
b) Determination of the peptides identified in a) that are predicted to be presented by any recipient's MHC class II HLA molecule;
c) Identification of peptides from all selected HLA molecules of the recipient;
d) Determination of the peptides identified in c) that are predicted to be presented by any recipient's MHC class II HLA molecule;
**wherein**
e) any peptide determined in b) that is not included in the set of peptides in d) is characterized as a PIRCHE II;
**characterised in that**
f) steps a) to d) are performed on information stored in a graph data structure,
**wherein**
g) the graph data structure comprises:
- one or more HLA value entities,
- one or more peptide entities, and
- one or more relationships between said HLA value and peptide entities, wherein each entity is present in a single instance within the graph data structure and may exhibit one or more relationships to another entity, and wherein the HLA value entities have relationships to peptides, but not to other HLA entities, and peptides have relationships to HLA values, but not to other peptides.

2. Method according to the preceding claim, wherein the information stored in the graph data structure is an electronically-stored representation of biological data and/or corresponds to one or more biological entities.

3. Method according to any one of the preceding claims, **characterised in that** HLA matching between donor and recipient has been conducted in advance of carrying out the method of claim 1.

4. Method according to any one of the preceding claims, **characterised in that** any given HLA value entity "has" a number of peptides (from 1 to n), wherein each peptide of (derived from) any given HLA value entity has a relationship to said HLA value entity, wherein preferably the peptides of each HLA value entity are determined by the predicted proteasome cleavage of the amino acid sequence of the corresponding HLA molecule.

5. Method according to any one of the preceding claims, **characterised in that** any given HLA value entity "presents" a number of peptides (from 0 to n), wherein each peptide presented by any given HLA value entity has a relationship to said HLA value entity, wherein preferably the presentation of a peptide by an HLA value entity is determined by the predicted binding and presentation of said peptide by said HLA molecule, wherein preferably the likelihood of peptide presentation is expressed by the IC 50 score.

6. Method according to any one of the preceding claims, **characterised in that** the selected HLA molecules are the HLA loci A*, B*, C*, DRB1* and DQB1*, and optionally DPB1* and DRB3/4/5*.

7. System for selecting and/or screening donor material for transplantation, for example for selecting donor material with permissible mismatches from mismatched unrelated donors, comprising software modules adapted for HLA matching, and for performing the method for determining the numbers of PIRCHES according to the preceding claims, wherein said system comprises a graph data structure according to any one of the preceding claims.

8. System according to the preceding claim, **characterised in that** the information corresponding to donor HLA value entities is obtained from one or more donor profiles, wherein the donor profiles are preferably stored in an additional data structure, database, library and/or simulation of the peptide and/or HLA value entities of any given one or more theoretical or virtual donors, wherein preferably multiple donor profiles or donor types are stored in a donor data structure, database, library and/or simulation comprising essentially all possible donors for essentially all possible genotypes or possible combinations of HLA alleles and/or haplotypes.

9. System according to any one of claims 7-8, wherein said system enables searching with recipient information corresponding to a biological recipient in need of a transplantation against one or more donor profiles, wherein the result (outcome) of said system provides information on the one or more (preferably multiple) best-matched mismatched donor profiles, wherein preferably the one or more best-matched mismatched donor profiles are 9/10 mismatched donors with a lowest possible number of PIRCHES compared to other mismatched donors.

10. System according to claim 9, wherein information representing the frequency of any given combination of HLA alleles (haplotype or genotype) in any one or more human populations is incorporated in the donor profile, preferably wherein electronically stored HLA typing data for donor material typed with "low-" or "medium-" resolution HLA typing is converted via a computer-implemented method into a "high-" resolution-similar or analogous HLA typing status on the basis of the known population frequencies of any given combination of HLA alleles determined by said with "low-" or "medium-" resolution HLA typing, thereby enabling interrogation of HLA typing data previously obtained via "low-" or "medium-" resolution techniques using the method and/or system as described herein, the system preferably comprising a) conversion of HLA typing data, preferably potential donors, from "low-" or "medium-" resolution HLA typing to "high-" resolution data, b) HLA matching of said donor information with recipient HLA information and c) PIRCHE matching according to the method and/or system of any one of the preceding claims.

11. Use of the system according to any one of claims 7-10 for the identification of permissible HLA mismatches for solid organ transplantation material .

## Patentansprüche

1. Computerumgesetztes Verfahren zum Bestimmen der Anzahl von vorhergesagten indirekt anerkannten HLA-Epitopen (PIRCHES) zwischen einem oder mehreren Spendern und einem oder mehreren Empfängern von festem Organtransplantationsmaterial, wobei die PIRCHES spenderspezifische von HLA abgeleitete Peptide aus einem nicht übereinstimmenden Spender-HLA-Allel sind und vorausgesagt wird, dass sie durch ein beliebiges HLA-Molekül der MHC-Klasse II präsentiert werden, wobei das Verfahren Folgendes umfasst:
a) Identifizierung von Peptiden aus nicht übereinstimmenden HLA-Molekülen des Spenders;
b) Bestimmung der Peptide, die in a) identifiziert wurden, von denen vorhergesagt wird, dass sie durch ein beliebiges der HLA-Moleküle der MHC-Klasse II des Empfängers präsentiert werden;
c) Identifizierung von Peptiden von allen ausgewählten HLA-Molekülen des Empfängers;
d) Bestimmung der Peptide, die in c) identifiziert sind, von denen vorhergesagt wird, dass sie durch ein beliebiges der HLA-Moleküle der MHC-Klasse II des Empfängers präsentiert werden;
**wobei**
e) ein beliebiges Peptid, das in b) bestimmt ist, das nicht in dem Satz von Peptiden in d) beinhaltet ist, als ein PIRCHE II **gekennzeichnet ist;**
**dadurch gekennzeichnet, dass**
f) die Schritte a) bis d) nach Informationen durchgeführt werden, die in einer Diagrammdatenstruktur gespeichert sind, **wobei**
g) die Diagrammdatenstruktur Folgendes umfasst:
- eine oder mehrere HLA-Werteinheiten,
- eine oder mehrere Peptideinheiten, und
- eine oder mehrere Beziehungen zwischen dem HLA-Wert und den Peptideinheiten, wobei jede Einheit in einer einzelnen Instanz innerhalb der Diagrammdatenstruktur vorhanden ist und eine oder mehrere Beziehungen zu einer anderen Einheit aufzeigen kann und wobei die HLA-Werteinheiten Beziehungen zu Peptiden, jedoch nicht zu anderen HLA-Einheiten, aufweisen und Peptide Beziehungen zu HLA-Werten, jedoch nicht zu anderen Peptiden, aufweisen.

2. Verfahren nach dem vorhergehenden Anspruch, wobei die Informationen, die in der Diagrammdatenstruktur gespeichert sind, eine elektronisch gespeicherte Darstellung von biologischen Daten sind und/oder einer oder mehreren biologischen Einheiten entsprechen.

3. Verfahren nach einem der vorhergehenden Ansprüche, **dadurch gekennzeichnet, dass** die HLA-Zuordnung zwischen Spender und Empfänger vor dem Ausführen des Verfahrens nach Anspruch 1 durchgeführt wurde.

4. Verfahren nach einem der vorhergehenden Ansprüche, **dadurch gekennzeichnet, dass** eine bestimmte HLA-Werteinheit eine Anzahl von Peptiden (von 1 bis n) "aufweist", wobei jedes Peptid von (abgeleitet von) einer beliebigen bestimmten HLA-Werteinheit eine Beziehung zu der HLA-Werteinheit aufweist, wobei vorzugsweise die Peptide von jeder HLA-Werteinheit durch die vorhergesagte Proteasomspaltung der Aminosäuresequenz des entsprechenden HLA-Moleküls bestimmt sind.

5. Verfahren nach einem der vorhergehenden Ansprüche, **dadurch gekennzeichnet, dass** eine beliebige bestimmte HLA-Werteinheit eine Anzahl von Peptiden (von 0 bis n) "präsentiert", wobei jedes Peptid, das durch eine beliebige bestimmte HLA-Werteinheit präsentiert ist, eine Beziehung zu der HLA-Werteinheit aufweist, wobei vorzugsweise die Präsentation eines Peptids durch eine HLA-Werteinheit durch die vorhergesagte Bindung und Präsentation des Peptids durch das HLA-Molekül bestimmt ist, wobei vorzugsweise die Wahrscheinlichkeit der Peptidpräsentation durch den IC50-Wert ausgedrückt ist.

6. Verfahren nach einem der vorhergehenden Ansprüche, **dadurch gekennzeichnet, dass** die ausgewählten HLA-Moleküle die HLA-Loci A*, B*, C*, DRB1* und DQB1* und wahlweise DPB1* und DRB3/4/5* sind.

7. System zum Auswählen und/oder Überprüfen von Spendermaterial zur Transplantation, zum Beispiel zum Auswählen von Spendermaterial mit erlaubten Nichtübereinstimmungen von nicht übereinstimmenden nicht verwandten Spendern, das Softwaremodule umfasst, die zum HLA-Zuordnen angepasst sind, und zum Durchführen des Verfahrens zum Bestimmen der Anzahl von PIRCHES gemäß den vorhergehenden Ansprüchen, wobei das System eine Diagrammdatenstruktur nach einem der vorhergehenden Ansprüche umfasst.

8. System nach dem vorhergehenden Anspruch, **dadurch gekennzeichnet, dass** die Informationen, die den Spender-HLA-Werteinheiten entsprechen, aus einem oder mehreren Spenderprofilen erlangt sind, wobei die Spenderprofile vorzugsweise in einer zusätzlichen Datenstruktur, Datenbank, Bibliothek und/oder Simulation des Peptids und/oder der HLA-Werteinheiten eines beliebigen bestimmten einen oder mehreren theoretischen oder virtuellen Spendern gespeichert sind, wobei vorzugsweise mehrere Spenderprofile oder Spendertypen in einer Spenderdatenstruktur, -datenbank, -bibliothek und/oder - simulation gespeichert sind, die im Wesentlichen alle möglichen Spender für im Wesentlichen alle möglichen Genotypen oder möglichen Kombinationen von HLA-Allelen und/oder Haplotypen umfasst.

9. System nach einem der Ansprüche 7-8, wobei das System ein Durchsuchen mit Empfängerinformationen, die einem biologischen Empfänger mit Bedarf an einer Transplantation entsprechen, in einem oder mehreren Spenderprofilen ermöglicht, wobei das Ergebnis (der Ausgang) des Systems Informationen über das eine oder die mehreren (vorzugsweise mehreren) am besten übereinstimmenden nicht übereinstimmenden Spenderprofile bereitstellt, wobei vorzugsweise das eine oder die mehreren am besten übereinstimmenden nicht übereinstimmenden Spenderprofile 9/10 nicht übereinstimmende Spender mit einer geringstmöglichen Anzahl von PIRCHES im Vergleich zu anderen nicht übereinstimmenden Spendern sind.

10. System nach Anspruch 9, wobei Informationen, die die Häufigkeit von einer beliebigen bestimmten Kombination von HLA-Allelen (Haplotyp oder Genotyp) in einer beliebigen einen oder mehreren menschlichen Populationen darstellen, in das Spenderprofil aufgenommen sind, vorzugsweise wobei elektronisch gespeicherte HLA-Typisierungsdaten für Spendermaterial, das mit "Niedrig-" oder "Mittel-" Auflösungs-HLA-Typisierung typisiert ist, über ein computerumgesetztes Verfahren in einen "Hoch-" Auflösungs-ähnlichen oder -analogen HLA-Typisierungsstatus umgewandelt sind, und zwar auf Basis der bekannten Populationshäufigkeiten einer beliebigen bestimmten Kombination von HLA-Allelen, die durch die "Niedrig-" oder "Mittel-" Auflösungs-HLA-Typisierung bestimmt ist, wodurch eine Abfrage der HLA-Typisierungsdaten ermöglicht wird, die zuvor über "Niedrig-" oder "Mittel-" Auflösungstechniken erlangt wurden, und zwar unter Verwendung des Verfahrens und/oder Systems wie hierin beschrieben, wobei das System vorzugsweise a) eine Umwandlung von HLA-Typisierungsdaten, vorzugweise mögliche Spender, von "Niedrig-" oder "Mittel-" Auflösungs-HLA-Typisierung zu "Hoch-" Auflösungsdaten, b) eine HLA-Zuordnung von bestimmten Spenderinformationen zu Empfänger-HLA-Informationen und c) eine PIRCHE-Zuordnung gemäß dem Verfahren und/oder System nach einem der vorhergehenden Ansprüche umfasst.

11. Verwendung des Systems nach einem der Ansprüche 7-10 zur Identifizierung von erlaubten HLA-Nichtübereinstimmungen für festes Organtransplantationsmaterial.

## Revendications

1. Procédé mis en œuvre par ordinateur pour déterminer les nombres d'épitopes HLA reconnus indirectement prédits (PIRCHES) entre un ou plusieurs donneurs et un ou plusieurs receveurs de matériel solide de transplantation d'organes, dans lequel lesdits PIRCHES sont des peptides dérivés de HLA spécifiques au donneur provenant d'un allèle HLA de donneur non apparié et il est prédit qu'ils soient présentés par toute molécule HLA de classe II du CMH, dans lequel ledit procédé comprend
a) l'identification de peptides à partir de molécules HLA non appariées du donneur ;
b) la détermination des peptides identifiés en a) dont il est prédit qu'ils soient présentés par toute molécule HLA de classe II du CMH du receveur ;
c) l'identification de peptides à partir de toutes les molécules HLA sélectionnées du receveur ;
d) la détermination des peptides identifiés en c) dont il est prédit qu'ils soient présentés par toute molécule HLA de classe II du CMH du receveur ;
**dans lequel**
e) tout peptide déterminé en b) qui n'est pas inclus dans l'ensemble de peptides en d) est caractérisé comme un PIRCHE II ;
**caractérisé en ce que**
f) les étapes a) à d) sont effectuées sur des informations stockées dans une structure de données de graphe,
**dans lequel**
g) la structure de données de graphe comprend :
- une ou plusieurs entités de valeur HLA,
- une ou plusieurs entités de peptide, et
- une ou plusieurs relations entre ladite valeur HLA et des entités peptidiques, dans lequel chaque entité est présente dans une instance unique à l'intérieur de la structure de données de graphe et peut présenter une ou plusieurs relations avec une autre entité, et dans lequel les entités de valeur HLA ont des relations avec des peptides, mais pas avec d'autres entités HLA, et des peptides ont des relations avec des valeurs HLA, mais pas avec d'autres peptides.

2. Procédé selon la revendication précédente, dans lequel les informations stockées dans la structure de données de graphe sont une représentation stockée électroniquement de données biologiques et/ou correspondent à une ou plusieurs entités biologiques.

3. Procédé selon l'une quelconque des revendications précédentes, **caractérisé en ce que** l'appariement HLA entre le donneur et le receveur a été réalisé avant la mise en œuvre du procédé selon la revendication 1.

4. Procédé selon l'une quelconque des revendications précédentes, **caractérisé en ce que** toute entité de valeur HLA donnée « a » un nombre de peptides (de 1 à n), dans lequel chaque peptide de (dérivé de) toute entité de valeur HLA donnée a une relation avec ladite entité de valeur HLA, dans lequel de préférence les peptides de chaque entité de valeur HLA sont déterminés par le clivage du protéasome prédit de la séquence d'acides aminés de la molécule HLA correspondante.

5. Procédé selon l'une quelconque des revendications précédentes, **caractérisé en ce que** toute entité de valeur HLA donnée « présente » un certain nombre de peptides (de 0 à n) , dans lequel chaque peptide présenté par toute entité de valeur HLA donnée a une relation avec ladite entité de valeur HLA, dans lequel de préférence la présentation d'un peptide par une entité de valeur HLA est déterminée par la liaison prédite et la présentation dudit peptide par ladite molécule HLA, dans lequel de préférence la probabilité de présentation de peptide est exprimée par le score CI 50.

6. Procédé selon l'une quelconque des revendications précédentes, **caractérisé en ce que** les molécules HLA sélectionnées sont les loci HLA A*, B*, C*, DRB1* et DQB1*, et éventuellement DPB1* et DRB3/4/5*.

7. Système de sélection et/ou de criblage de matériel de transplantation du donneur, par exemple pour sélectionner du matériel de donneur avec des mésappariements autorisés de donneurs non liés non appariés, comprenant des modules logiciels conçus pour un appariement HLA, et pour mettre en œuvre le procédé de détermination des nombres de PIRCHES selon les revendications précédentes, dans lequel ledit système comprend une structure de données de graphe selon l'une quelconque des revendications précédentes.

8. Système selon la revendication précédente, **caractérisé en ce que** les informations correspondant à des entités de valeur HLA du donneur sont obtenues à partir d'un ou plusieurs profils de donneurs, dans lequel les profils de donneurs sont de préférence stockés dans une structure de données, base de données, bibliothèque et/ou simulation supplémentaire du peptide et/ou des entités de valeur HLA de n'importe quels un ou plusieurs donneurs théoriques ou virtuels donnés, dans lequel de préférence de nombreux profils de donneurs ou types de donneurs sont stockés dans une structure de données, base de données, bibliothèque et/ou simulation de donneur comprenant pratiquement tous les donneurs possibles pour pratiquement tous les génotypes possibles ou toutes les combinaisons possibles d'allèles et/ou d'haplotypes HLA.

9. Système selon l'une quelconque des revendications 7 à 8, dans lequel ledit système permet d'effectuer des recherches avec des informations de receveur correspondant à un receveur biologique ayant besoin d'une transplantation par rapport à un ou plusieurs profils de donneur, dans lequel le résultat (bilan) dudit système fournit des informations sur les un ou plusieurs (de préférence nombreux) profils de donneurs non appariés les mieux appariés, dans lequel de préférence les un ou plusieurs profils de donneurs non appariés les mieux appariés sont 9/10 donneurs non appariés avec un nombre le plus bas possible de PIRCHES par rapport à d'autres donneurs non appariés.

10. Système selon la revendication 9, dans lequel des informations représentant la fréquence de toute combinaison donnée d'allèles HLA (haplotype ou génotype) dans n'importe quelles une ou plusieurs populations humaines sont incorporées dans le profil du donneur, de préférence dans lequel les données de typage HLA stockées électroniquement pour le matériel de donneur typées avec un typage HLA « basse » ou « moyenne » résolution sont converties via une méthode mise en œuvre par ordinateur dans un état de typage HLA « haute » résolution similaire ou analogue sur la base des fréquences de population connues de toute combinaison donnée d'allèles HLA déterminés par ledit typage HLA « basse » ou « moyenne » résolution, permettant ainsi l'interrogation de données de typage HLA obtenues précédemment via des techniques « basse » ou « moyenne » résolution à l'aide du procédé et/ou du système tels que décrits ici, le système comprenant de préférence a) la conversion de données de typage HLA, de préférence de donneurs potentiels, du typage HLA « basse » ou « moyenne » résolution en données « haute » résolution, b) l'appariement HLA desdites informations de donneur avec des informations HLA de receveur et c) l'appariement PIRCHE selon le procédé et/ou système d'une quelconque des revendications précédentes.

11. Utilisation du système selon l'une quelconque des revendications 7 à 10 pour l'identification de mésappariements HLA autorisés pour un matériel solide de transplantation d'organes.
